# EUROPEAN PATENT APPLICATION

(11) **EP 4 772 208 A2**
(43) Date of publication of application: **08.07.2026**
(21) Application number: 26179713.8
(22) Date of filing: 29.12.2022
(51) Int. Cl.: A61M 1/36

(54) **DISPOSABLE SET, APPARATUS AND PROCEDURE OF BLOOD RECIRCULATION IN AN EXTRACORPOREAL BLOOD TREATMENT APPARATUS**

(62) Divisional of application: 22307062.4
(71) Applicant: Gambro Lundia AB, 226 43 Lund (SE)
(72) Inventor: POUCHOULIN, Dominique, F-01390 Tramoyes (FR); FRUGIER, Alain, 69380 Chazay d'Azergues (FR)
(74) Representative: PGA S.p.A., Milano, Succursale di Lugano

(57) **Abstract**

Disposable set and extracorporeal blood treatment apparatus comprising a blood circuit including a filtration unit, a blood withdrawal line, a blood return line, a blood pump tract on the blood withdrawal line or on the blood return line and configured to be engaged to a blood pump of the extracorporeal blood treatment apparatus to promote blood flow in the blood circuit. The blood circuit further comprises an infusion connected to both the blood withdrawal line at a first site, and to the return line at a second site. The blood circuit is configurable in a loop recirculation circuit including the filtration unit, the blood pump tract, a part of the blood withdrawal line, a part of the blood return line and a recirculation portion of the infusion line interposed between the first site and the second site.

## Description

### FIELD OF THE INVENTION

The present invention relates to a disposable set for an extracorporeal blood treatment apparatus configured to allow for fluid/blood recirculation in loop, for example when an alarm is issued stopping the blood pump, and the treatment has to be (e.g., temporarily) interrupted. The invention also relates to an apparatus for extracorporeal blood treatment and to a procedure to perform fluid/blood recirculation in loop in a disposable set of an extracorporeal blood treatment apparatus. The extracorporeal blood treatment apparatus of the present invention may perform dialysis treatments such as hemodialysis (HD) treatments, hemofiltration (HF) treatments, hemodiafiltration (HDF) treatments, or ultrafiltration (UF) treatments, as well as Continuous Renal Replacement Therapy (CRRT) treatments; the apparatus may also be configured for ECCOZR or oxygenation treatments, as well as for adsorption, hemoperfusion, liver treatments, or plasmapheresis.

### BACKGROUND

The kidneys fulfil many functions, including the removal of water, the excretion of catabolites (or waste from the metabolism, for example urea and creatinine), the regulation of the concentration of the electrolytes in the blood (e.g. sodium, potassium, magnesium, calcium, bicarbonates, phosphates, chlorides) and the regulation of the acid/base equilibrium within the body, which is obtained in particular by the removal of weak acids and by the production of ammonium salts. In individuals who have lost (temporarily or permanently) the use of their kidneys, since these excretion and regulation mechanisms no longer work, the body accumulates water and waste from the metabolism and exhibits an excess of electrolytes, as well as, in general, acidosis, the pH of the blood plasma shifting downwards, below 7.35 (the blood pH normally varies within narrow limits of between 7.35 and 7.45). As mentioned, in order to overcome renal dysfunction, resort is conventionally made to a blood treatment involving extracorporeal circulation through an exchanger having a semipermeable membrane (dialyzer) in which the patient's blood is circulated on one side of the membrane and a dialysis liquid, comprising the main electrolytes of the blood in concentrations close to those in the blood of a healthy subject, is circulated on the other side. Furthermore, a pressure difference is created between the two compartments of the dialyzer which are delimited by the semipermeable membrane, so that a fraction of the plasma fluid passes by ultrafiltration through the membrane into the compartment containing the dialysis liquid. The blood treatment which takes place in a dialyzer as regards waste from the metabolism and electrolytes results from two mechanisms of molecular transport through the membrane. On the one hand, the molecules migrate from the liquid where their concentration is higher to the liquid where their concentration is lower. This is diffusive transport. On the other hand, certain catabolites and certain electrolytes are entrained by the plasma fluid which filters through the membrane under the effect of the pressure difference created between the two compartments of the exchanger. This is convective transport. Three of the abovementioned functions of the kidney, namely the removal of water, the excretion of catabolites and the regulation of the electrolytic concentration of the blood, are therefore performed in a conventional blood treatment device by the combination of dialysis and blood filtration (this combination is referred to as hemodiafiltration). To perform one or more of the above described treatments, the extracorporeal blood treatment equipment may comprise a disposable set including a plurality of lines for delivering fluid directly to the patient or into the extracorporeal blood circuit. The disposable set for extracorporeal blood treatment commonly comprises a fluid circuit including fluid lines, blood lines and a filtration unit. The disposable set is usually engaged to a front panel of an extracorporeal blood treatment apparatus for delivering any one of the above mentioned treatments. In case of extracorporeal blood circulation, one relevant concern includes reducing the risk of blood clotting within the blood lines of the disposable set.

However, during an extracorporeal blood treatment, it may be necessary to stop the blood pump (i.e., stop the extracorporeal blood circulation) when the extracorporeal blood is still in the blood circuit of the apparatus, for example, when an alarm is issued requiring to temporally suspend the treatment to allow the operator to fix the problem. In such a situation, the blood remains stationary in the blood lines increasing the risk of blood clotting both in the blood lines and in the filtration unit. Blood clotting risk increases with passing time, therefore, the operator should be able to resume the treatment in the shortest time possible. In such alarm situations with the blood pump stopped, stressful situations are caused on the operators, particularly the less experienced ones.

In case blood clots in the lines and/or in the filtration unit, the disposable set has to be disposed and replaced with a new one. This leads to substantive loss of blood from the patient and determines an increase of the treatment costs and time.

### AIM OF THE INVENTION

The aim of the invention is therefore to at least partially solve one or more of the drawbacks and/or limitations described above. A first aim is to reduce the risk of clotting in the extracorporeal blood circuit by prevention of blood stagnation. A further object is to more easily address alarms that stop the blood pump and thus induce a risk of circuit clotting if troubleshooting is delayed, preventing, or at least reducing, the risk of blood clotting in the lines of the disposable set. A further aim is to give the practitioner more time (thus also reducing stress) for troubleshooting, such as fixing treatment or patient issues and/or resetting apparatus parameters, during the temporary interruption in the treatment. An additional aim is to improve the life time of the disposable set, which is particularly relevant in CRRT therapies. A further aim is to avoid the blood volume housed in the disposable set to be discarded, which results in blood loss for the patient and the disposal of the circuit, thus also reducing waste during extracorporeal blood treatments. An auxiliary aim is to allow for recirculation in the blood withdrawal line to the maximum extent possible so to avoid blood stagnation in a long portion of the blood lines.

These objects and more, which will appear more from the following description, are substantially achieved by a disposable set, an apparatus, and a recirculation procedure in accordance with one or more of the following claims and/or aspects. Not all aims may be achieved by each and every embodiment of the invention.

### SUMMARY SECTION

The disposable set of the invention is according to the feature combination of claim 1. The apparatus of the invention is according to the feature combination of claim 13.

A first aspect refers to an extracorporeal blood treatment apparatus including:
- one or more pumps (21, 25, 26, 54, 65, 75) comprising a blood pump (21),
- one or more intercepting elements (20, 27, 59, 70),
- a disposable set (100) comprising:
   ➢ a filtration unit (2) having a blood inlet (2a) and a blood outlet (2b);
   ➢ a blood circuit (17) including:
      ▪ a blood withdrawal line (6) extending between a first end (6a) connected to the blood inlet (2a) of the filtration unit (2), and a second end (6b) for connection to a patient (P);
      ▪ a blood return line (7) extending between a first end (7a) connected to the blood outlet (2b) of the filtration unit (2), and a second end (7b) for connection to the patient;
      ▪ a blood pump tract (6p) located on the blood circuit (17) and engaged to the blood pump (21) of the extracorporeal blood treatment apparatus to promote blood flow in the blood circuit,
   ➢ an infusion line (63) extending from a first end (63a) for connection to an infusion fluid source (64) and branching off at a branch site (67) towards:
      ▪ a second end (63b) of the infusion line (63) in direct or indirect fluid communication with the blood withdrawal line (6) at a first site (31), the first site (31) being interposed between the second end (6b) of the blood withdrawal line (6) and the blood pump tract (6p); and
      ▪ a third end (63c) of the infusion line (63) in direct or indirect fluid communication with the blood return line (7) at a second site (32), the second site (32) being interposed between the blood pump tract (6p) and the second end of the blood return line (7);
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63), the control unit (12) being configured to define at least the following flow circuits:
   ➢ a treatment flow circuit, wherein the one or more pumps (21, 25, 26, 54, 65, 75) and the one or more intercepting elements (20, 27, 59, 70) are configured to allow blood to flow from the second end (6b) of the blood withdrawal line (6) to the filtration unit (2) and from the filtration unit (2) to the second end (7b) of the blood return line (7); and
   ➢ a loop recirculation circuit (11), wherein the one or more pumps (21, 25, 26, 54, 65, 75) and the one or more intercepting elements (20, 27, 59, 70) are configured to allow blood or a fluid to continuously flow in a loop circulation,
wherein the loop recirculation circuit (11) includes:
- the filtration unit (2);
- the blood pump tract (6p);
- a portion of the blood withdrawal line (6) interposed between the first site (31) of the blood withdrawal line (6) and the blood inlet (2a) of the filtration unit (2);
- a portion of the blood return line (7) interposed between the blood outlet (2b) of the filtration unit (2) and the second site (32) of the blood return line (7);
- a recirculation portion (66) of the infusion line (63) downstream the branch site (67) and interposed between the first site (31) and the second site (32).

In a further aspect according to the previous aspect, the control unit (12) is configured to perform a recirculation procedure comprising the steps of:
- defining the loop recirculation circuit (11) by at least:
   ➢ commanding the one or more intercepting elements (20, 27, 59, 70) to put in fluid communication, the first site (31) with the second site (32) through the recirculation portion (66) of the infusion line (63);
- controlling the blood pump (21) to determine blood flow within the loop recirculation circuit (11) of the blood circuit (17) of the disposable set,
   in particular wherein the one or more intercepting elements (20, 27, 59, 70) comprise an intercepting element (70) acting on the infusion line (63) and movable between:
   ➢ a recirculation position, wherein the intercepting element (70) is configured to allow fluid communication between the first site (31) and the second site (32) through the recirculation portion (63) of the infusion line (63);
   ➢ a lock position, wherein the intercepting element (70) is configured to prevent fluid communication between the first site (31) and the second site (32) through the recirculation portion (63) of the infusion line (63);
and wherein, in the recirculation procedure, the control unit (12) is configured to switch the intercepting element (70) to the recirculation position.

In a further aspect according to the previous aspect, before starting the recirculation procedure, the control unit (12) is configured to:
a. stop the blood pump (21);
b. prior switching the intercepting element (70) to the recirculation position, operate on the blood circuit (17) to reduce a pressure difference between portions of the blood circuit (17) across the blood pump (21);
in particular wherein to reduce the pressure difference, the control unit (12) is configured to perform at least one of the following actions:
i. commanding the one or more intercepting elements (20, 27, 59, 70) from a close to an open position to switch from preventing to allowing fluid passage in the blood withdrawal line (6) between the second end (6b) of the blood withdrawal line (6) and the first site (31) and/or in the blood return line (7) between the second site (32) and the second end (7b) of the blood return line (7);
ii. infusing an infusion fluid into the blood circuit (17) to adjust at least one of an access pressure and a return pressure as to bring them to a closer value, for example to a pressure difference of less than 100 mmHg.

In a further aspect according to any one of the previous aspects, one or more pumps (21, 25, 26, 54, 65, 75) comprises an infusion pump (65) active on the infusion pump tract (63p) of the infusion line, wherein, in the recirculation procedure, the control unit (12) is configured to:
c. keep said infusion pump (65) inactive, in particular the infusion pump (65) being an occlusive pump, such as a peristaltic pump;
d. stop any ultrafiltration of fluids from a primary chamber (3) towards a secondary chamber (4) of the filtration unit (2);
e. recirculate blood in the loop recirculation circuit (11) to prevent blood from clotting;
f. optionally run the blood pump (21) at a speed different from a speed of the blood pump (21) during the extracorporeal blood treatment, in particular, during the recirculation procedure, the control unit (12) is configured to run the blood pump (21) in a same direction as during the extracorporeal blood treatment.

In a further aspect according to any one of the previous aspects, at an end of the recirculation procedure, the control unit (12) is configured to:
g. move an intercepting element (70) acting on the infusion line (63) to a lock position allowing fluid flow in a second tract (68) extending from the branch site (67) up to the second end (63b) of the infusion line (63) and blocking a fluid flow in a third tract (69) extending from the branch site (67) up to the third end (63c) of the infusion line (63) to prevent fluid communication between the first site (31) and the second site (32) through the recirculation portion (66);
h. rinse the blood in the second tract (68) with a fluid from the infusion line (63).

In a 2^{nd} independent aspect a disposable set (100) for an extracorporeal blood treatment apparatus (1) is provided comprising:
- a filtration unit (2) having a blood inlet (2a) and a blood outlet (2b);
- a blood circuit (17) including:
   ➢ a blood withdrawal line (6) extending between a first end (6a) connected to the blood inlet (2a) of the filtration unit (2), and a second end (6b) for connection to a patient (P);
   ➢ a blood return line (7) extending between a first end (7a) connected to the blood outlet (2b) of the filtration unit (2), and a second end (7b) for connection to the patient;
   ➢ a blood pump tract (6p) located on the blood circuit (17) and configured to be engaged to a blood pump (21) of the extracorporeal blood treatment apparatus to promote blood flow in the blood circuit,
- an infusion line (63) extending from a first end (63a) for connection to an infusion fluid source (64) and branching off at a branch site (67) towards:
   ➢ a second end (63b) of the infusion line (63) in direct or indirect fluid communication with the blood withdrawal line (6) at a first site (31), the first site (31) being interposed between the second end (6b) of the blood withdrawal line (6) and the blood pump tract (6p); and
   ➢ a third end (63c) of the infusion line (63) in direct or indirect fluid communication with the blood return line (7) at a second site (32), the second site (32) being interposed between the blood pump tract (6p) and the second end of the blood return line (7);

wherein the blood circuit is designed to allow fluid flow in a loop recirculation circuit (11) including:
   - the filtration unit (2);
   - the blood pump tract (6p);
   - a portion of the blood withdrawal line (6) interposed between the first site (31) of the blood withdrawal line (6) and the blood inlet (2a) of the filtration unit (2);
   - a portion of the blood return line (7) interposed between the blood outlet (2b) of the filtration unit (2) and the second site (32) of the blood return line (7);
   - a recirculation portion (66) of the infusion line (63) downstream the branch site (67) and interposed between the first site (31) and the second site (32);
the loop recirculation circuit (11) being configured to allow blood or a fluid, at least in a recirculation condition, to continuously flow in a loop circulation.

In a 3^{rd} independent aspect a method of recirculating blood in an extracorporeal blood treatment apparatus (1) is provided, wherein the apparatus (1) includes:
- one or more pumps (21, 25, 26, 54, 65, 75) comprising a blood pump (21),
- one or more intercepting elements (20, 27, 59, 70),
- a disposable set (100), particularly in accordance with any one of the previous claims, comprising:
   ➢ a filtration unit (2) having a blood inlet (2a) and a blood outlet (2b);
   ➢ a blood circuit (17) including:
      ▪ a blood withdrawal line (6) extending between a first end (6a) connected to the blood inlet (2a) of the filtration unit (2), and a second end (6b) for connection to a patient (P);
      ▪ a blood return line (7) extending between a first end (7a) connected to the blood outlet (2b) of the filtration unit (2), and a second end (7b) for connection to the patient;
      ▪ a blood pump tract (6p) located on the blood circuit (17) and engaged to the blood pump (21) of the extracorporeal blood treatment apparatus to promote blood flow in the blood circuit,
   ➢ an infusion line (63) extending from a first end (63a) for connection to an infusion fluid source (64) and branching off at a branch site (67) towards:
      ▪ a second end (63b) of the infusion line (63) in direct or indirect fluid communication with the blood withdrawal line (6) at a first site (31), the first site (31) being interposed between the second end (6b) of the blood withdrawal line (6) and the blood pump tract (6p); and
      ▪ a third end (63c) of the infusion line (63) in direct or indirect fluid communication with the blood return line (7) at a second site (32), the second site (32) being interposed between the blood pump tract (6p) and the second end of the blood return line (7);
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63),

wherein the method of recirculating blood comprises switching from:
   ➢ a treatment flow circuit, wherein the one or more pumps (21, 25, 26, 54, 65, 75) and the one or more intercepting elements (20, 27, 59, 70) allow blood to flow from the second end (6b) of the blood withdrawal line (6) to the filtration unit (2) and from the filtration unit (2) to the second end (7b) of the blood return line (7); to
   ➢ a loop recirculation circuit (11), wherein the one or more pumps (21, 25, 26, 54, 65, 75) and the one or more intercepting elements (20, 27, 59, 70) allow blood or a fluid to continuously flow in a loop circulation,
wherein the loop recirculation circuit (11) includes:
   - the filtration unit (2);
   - the blood pump tract (6p);
   - a portion of the blood withdrawal line (6) interposed between the first site (31) of the blood withdrawal line (6) and the blood inlet (2a) of the filtration unit (2);
   - a portion of the blood return line (7) interposed between the blood outlet (2b) of the filtration unit (2) and the second site (32) of the blood return line (7);
a recirculation portion (66) of the infusion line (63) downstream the branch site (67) and interposed between the first site (31) and the second site (32).

In a 4^{th} independent aspect an extracorporeal blood treatment apparatus including:
- one or more pumps (21, 25, 26, 54, 65, 75) comprising a blood pump (21),
- one or more intercepting elements (20, 27, 59, 70),
- a disposable set (100) comprising:
   ➢ a filtration unit (2) having a blood inlet (2a) and a blood outlet (2b);
   ➢ a blood circuit (17) including:
      ▪ a blood withdrawal line (6) extending between a first end (6a) connected to the blood inlet (2a) of the filtration unit (2), and a second end (6b) for connection to a patient (P);
      ▪ a blood return line (7) extending between a first end (7a) connected to the blood outlet (2b) of the filtration unit (2), and a second end (7b) for connection to the patient;
      ▪ a blood pump tract (6p) located on the blood circuit (17) and engaged to the blood pump (21) of the extracorporeal blood treatment apparatus to promote blood flow in the blood circuit,
   ➢ a pre-blood-pump infusion line (51) extending from a first end (51a) for connection to a second fluid source (10), and a second end (51b) fluidly connected to the blood withdrawal line (6) between the second end (6b) of the blood withdrawal line (6) and the blood pump tract (6p);
   ➢ an infusion line (63) extending from a first end (63a) for connection to an infusion fluid source (64) and branching off at a branch site (67) towards:
      ▪ a second end (63b) of the infusion line (63) directly connected to the pre-blood-pump infusion line (51) and in fluid communication with the blood withdrawal line (6) at a first site (31), the first site (31) being interposed between the second end (6b) of the blood withdrawal line (6) and the blood pump tract (6p); and
      ▪ a third end (63c) of the infusion line (63) in direct or indirect fluid communication with the blood return line (7) at a second site (32), the second site (32) being interposed between the blood pump tract (6p) and the second end of the blood return line (7);
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63), the control unit (12) being configured to define at least the following flow circuits:
   ➢ a treatment flow circuit, wherein the one or more pumps (21, 25, 26, 54, 65, 75) and the one or more intercepting elements (20, 27, 59, 70) are configured to allow blood to flow from the second end (6b) of the blood withdrawal line (6) to the filtration unit (2) and from the filtration unit (2) to the second end (7b) of the blood return line (7); and
   ➢ a loop recirculation circuit (11), wherein the one or more pumps (21, 25, 26, 54, 65, 75) and the one or more intercepting elements (20, 27, 59, 70) are configured to allow blood or a fluid to continuously flow in a loop circulation,
wherein the infusion line (63) and the pre-blood-pump infusion line (51) are connected together and present a common infusion tract extending up to the first site (31) of the blood withdrawal line (6), wherein the loop recirculation circuit (11) includes:
- the filtration unit (2);
- the blood pump tract (6p);
- a portion of the blood withdrawal line (6) interposed between the first site (31) of the blood withdrawal line (6) and the blood inlet (2a) of the filtration unit (2);
- a portion of the blood return line (7) interposed between the blood outlet (2b) of the filtration unit (2) and the second site (32) of the blood return line (7);
- a recirculation portion (66) of the infusion line (63) downstream the branch site (67) and interposed between the first site (31) and the second site (32)
- the common infusion tract.

In a 5^{th} independent aspect a disposable set (100) for an extracorporeal blood treatment apparatus (1) is provided comprising:
- a filtration unit (2) having a blood inlet (2a) and a blood outlet (2b);
- a blood circuit (17) including:
   ➢ a blood withdrawal line (6) extending between a first end (6a) connected to the blood inlet (2a) of the filtration unit (2), and a second end (6b) for connection to a patient (P);
   ➢ a blood return line (7) extending between a first end (7a) connected to the blood outlet (2b) of the filtration unit (2), and a second end (7b) for connection to the patient;
   ➢ a blood pump tract (6p) located on the blood circuit (17) and configured to be engaged to a blood pump (21) of the extracorporeal blood treatment apparatus to promote blood flow in the blood circuit,
- a pre-blood-pump infusion line (51) extending from a first end (51a) for connection to a second fluid source (10), and a second end (51b) fluidly connected to the blood withdrawal line (6) between the second end (6b) of the blood withdrawal line (6) and the blood pump tract (6p);
- an infusion line (63) extending from a first end (63a) for connection to an infusion fluid source (64) and branching off at a branch site (67) towards:
   ➢ a second end (63b) of the infusion line (63) directly connected to the pre-blood-pump infusion line (51) and in fluid communication with the blood withdrawal line (6) at a first site (31), the first site (31) being interposed between the second end (6b) of the blood withdrawal line (6) and the blood pump tract (6p); and
   ➢ a third end (63c) of the infusion line (63) in direct or indirect fluid communication with the blood return line (7) at a second site (32), the second site (32) being interposed between the blood pump tract (6p) and the second end of the blood return line (7);

wherein the infusion line (63) and the pre-blood-pump infusion line (51) are connected together and present a common infusion tract extending up to the first site (31) of the blood withdrawal line (6), wherein the blood circuit is designed to allow fluid flow in a loop recirculation circuit (11) including:
   - the filtration unit (2);
   - the blood pump tract (6p);
   - a portion of the blood withdrawal line (6) interposed between the first site (31) of the blood withdrawal line (6) and the blood inlet (2a) of the filtration unit (2);
   - a portion of the blood return line (7) interposed between the blood outlet (2b) of the filtration unit (2) and the second site (32) of the blood return line (7);
   - a recirculation portion (66) of the infusion line (63) downstream the branch site (67) and interposed between the first site (31) and the second site (32);
   - the common infusion tract;
the loop recirculation circuit (11) being configured to allow blood or a fluid, at least in a recirculation condition, to continuously flow within in a loop circulation.

In the following depending aspects, features of the disposable set are provided which may be implemented in combination with the apparatus, disposable set, and method of the previous aspects.

In a 6^{th} aspect according to any one of the previous aspects, the loop recirculation circuit (11) is configured to allow fluid recirculation in a first direction from the first site (31) towards the filtration unit (2) and from the filtration unit (2) towards the second site (32) or in an second direction from the second site (32) towards the filtration unit (2) and from the filtration unit (2) towards the first site (31).

In a 7^{th} aspect according to any one of the previous aspects:
- the blood inlet (2a) of the filtration unit (2) is in bidirectional fluid communication with the recirculation portion (66) of the infusion line (63) through the first site (31); and
- the blood outlet (2b) of the filtration unit (2) is in bidirectional fluid communication with the recirculation portion (66) of the infusion line (63) through the second site (32).

In an 8^{th} aspect according to any one of the previous aspects, the second end (63b) of the infusion line (63) is directly connected to the blood withdrawal line (6) at a first site (31).

In a 9^{th} aspect according to any one of the previous aspects, the first site (31) comprises a fluid connector, in particular a three-way fluid connector, including:
- a blood inlet fluidly connected to the withdrawal line (6) and facing the second end (6b) of the blood withdrawal line (6);
- a blood outlet fluidly connected to the withdrawal line (6) and facing the first end (6a) of the blood withdrawal line (6), optionally facing the pump tract (6p) of the blood withdrawal line (6);
- an infusion inlet fluidly connected to the recirculation portion (66) of the infusion line (63), and in particular fluidly connected to the second end (63b) of the infusion line (63).

In a 10^{th} aspect according to any one of the previous aspects, the disposable set further comprises at least one pressure detecting site (48a; 49a) arranged on the blood withdrawal line (6) between the first site (31) and the filtration unit (2), in particular said at least one pressure detecting site (48a; 49a) being a pressure pod.

In a 11^{th} aspect according to any one of the previous aspects, the disposable set further comprises a pressure detecting site (48a) arranged on the blood withdrawal line (6) between the first site (31) and the blood pump tract (6p), in particular said pressure detecting site (48a) being a pressure pod.

In a 12^{th} aspect according to any one of the previous aspects, the disposable set further comprises a pressure detecting site (49a) arranged on the blood withdrawal line (6) between the blood pump tract (6p) and the filtration unit (2), in particular said pressure detecting site (49a) being a pressure pod.

In a 13^{th} aspect according to any one of the previous aspects, the disposable set further comprises a pressure detecting site (50a) arranged on the infusion line (63) between the branch site (67) and the second end (63b) of the infusion line (63).

In a 14^{th} aspect according to any one of the previous aspects:
- the pump tract (6p) is arranged on the blood withdrawal line (6), in particular interposed between the first site (31) and the first end (6a) of the blood withdrawal line (6);
   or
- the pump tract (6p) is arranged on the blood return line (7), in particular between the first end (7a) of the blood return line (7) and the second site (32).

In a 15^{th} aspect according to any one of the previous aspects, the first site (31) is arranged on the blood withdrawal line (6) upstream the blood pump tract (6p) with respect to a blood withdrawal direction (200) directed from the second end (6b) to the first end (6a) of the blood withdrawal line (6), in particular the first site (31) being arranged upstream a blood pump (21) of the extracorporeal blood treatment apparatus configured to act on the blood pump tract (6p) of the blood withdrawal line (6).

In a 16^{th} aspect according to any one of the previous aspects, the infusion line (63) extends from the first end (63a) of the infusion line (63) for a first tract (72), said first tract (72) branching at the branch site (67) in:
- a second tract (68) extending from the branch site (67) up to the second end (63b) of the infusion line (63);
   and
- a third tract (69) extending from the branch site (67) up to the third end (63c) of the infusion line (63),
   the recirculation portion (66) of the infusion line (63) comprising the second tract (68) and the third tract (69) of the infusion line (63).

In a 17^{th} aspect according to the previous aspect, the infusion line (63) comprises a respective infusion pump tract (65p) configured to be engaged by an infusion pump (65) of the extracorporeal blood treatment apparatus, said infusion pump tract (65p) being interposed between the first end (63a) and the branch site (67) of the infusion line (63).

In an 18^{th} aspect according to the previous aspect, the infusion pump tract (65p) of the infusion line (63) is arranged on the first tract (72) of the infusion line (63), in particular the infusion pump tract (65p) being part of the first tract (72) of the infusion line (63).

In a 19^{th} aspect according to any one of the previous aspects, the fluid source (64) comprises a pre-packaged bag containing a replacement solution.

In a 20^{th} aspect according to any one of the previous aspects, the third end (63c) of the infusion line (63) is directly connected to the blood return line (7) at the second site (32).

In a 21^{st} aspect according to any one of the previous aspects, the disposable set comprises a bubble trap (19) arranged on the return line (7) of the blood circuit (17), said bubble trap (19) being configured to remove bubbles from the blood during the extracorporeal blood treatment.

In a 22^{nd} aspect according to the previous aspect, the second site (32) is arranged at the bubble trap (19), the third end (63c) of the infusion line (63) being fluidly connected to the bubble trap (19), and wherein the loop recirculation circuit (11) further comprises said bubble trap (19).

In a 23^{rd} aspect according to any one of the previous two aspects, the bubble trap (19) comprises:
- a blood inlet connected to the receiving portion of the blood return line (7), the receiving portion of the return line (7) being interposed between the outlet (2b) of the filtration unit (2) and said blood inlet of the bubble trap (19);
- a blood outlet connected to a secondary tract of the blood return line (7), the secondary tract of the blood return line (7) being interposed between said blood outlet of the bubble trap (19) and the second end (7b) of the return line (7);
- an auxiliary connection connected to the third end (63c) of the infusion line (63).

In a 24^{th} aspect according to any one of the previous aspects, the first site (31) is closer to the second end (6b) of the blood withdrawal line (6) than to the first end (6a) of the blood withdrawal line (6).

In a 25^{th} aspect according to any one of the previous aspects, the disposable set further comprises a pre-blood-pump infusion line (51) extending from a first end (51a) for connection to a second fluid source (10), and a second end (51b) fluidly connected to the blood withdrawal line (6).

In a 26^{th} aspect according to the previous aspect, the second end (51b) of the pre-blood-pump infusion line (51) is interposed between the second end (6b) of the blood withdrawal line (6) and the blood pump tract (6p) and/or between the second end (6b) of the blood withdrawal line (6) and the first site (31).

In a 27^{th} aspect according to any one of the previous two aspects, the pre-blood-pump infusion line (51) comprises a respective PBP infusion pump tract (51p) configured to be engaged by a PBP infusion pump (54), in particular said PBP infusion pump (54) being configured to promote infusion of a substance through said pre-blood-pump infusion line (51).

In a 28^{th} aspect according to any one of the previous two aspects, the second end (63b) of the infusion line (63) is directly connected to the pre-blood-pump infusion line (51), in particular downstream a PBP infusion pump tract (51p) of the pre-blood-pump infusion line (51) configured to be engaged by a PBP infusion pump (54).

In a 29^{th} aspect according to any one of the previous three aspects, the infusion line (63) and the pre-blood-pump infusion line (51) are connected together and present a common infusion tract extending up to the first site (31) of the blood withdrawal line (6), the loop recirculation circuit (11) comprising said common infusion tract.

In a 30^{th} aspect according to any one of the previous four aspects, the disposable set further comprises a pressure detecting site (52a) arranged on the pre-blood-pump infusion line (51) between a PBP infusion pump tract (51p) of the pre-blood-pump infusion line (51) and the second end (51b) of the pre-blood-pump infusion line (51), in particular said pressure detecting site (52a) being a pressure pod.

In a 31^{st} aspect according to the previous aspect and aspect 27, the pressure detecting site (52a) is arranged between the PBP infusion pump tract (51p) and the second end (63b) of the infusion line (63).

In a 32^{nd} aspect according to any one of the previous six aspects, the pre-blood-pump infusion line (51) is an anticoagulant infusion line, in particular the second fluid source (10) comprising a PBP bag containing citrate and/or citric acid.

In a 33^{rd} aspect according to any one of the previous aspects, the filtration unit (2) comprises:
- a primary chamber (3) fluidly connected to the blood circuit through the blood inlet and the blood outlet of the filtration unit (2),
- a secondary chamber (4) fluidly connected or connectable to a fluid circuit (16), said secondary chamber (4) being configured to receive dialysis fluid and/or to remove effluent fluid,
   and wherein the primary chamber (3) is separated from the secondary chamber (4) by a semi-permeable membrane (5).

In a 34^{th} aspect according to any one of the previous aspects, the disposable set further comprises a fluid circuit (16) including:
- an effluent fluid line (13) extending between a first end for connection to a discharge unit, such as a collection container (62), and a second end fluidly connected to a fluid outlet of a secondary chamber (4) of the filtration unit (2), wherein said effluent fluid line (13) comprises an effluent pump tract (13p) configured to be engaged to an effluent pump (26) of the extracorporeal blood treatment apparatus to promote flow of effluent fluid from the fluid outlet of the secondary chamber (4) towards the discharge unit;
- optionally a fluid supply line (8) extending between a first end for connection to a dialysis fluid source (14), and a second end fluidly connected to a fluid inlet of a secondary chamber (4) of the filtration unit (2), wherein said fluid supply line (8) comprises a supply pump tract (8p) configured to be engaged to a supply pump (25) of the extracorporeal blood treatment apparatus to promote dialysis fluid towards the fluid inlet of the secondary chamber (4) of the filtration unit (2).

In a 35^{th} aspect according to the previous aspect, the fluid supply line (8) splits in a post infusion tract (58) extending up to the return line (7) so that the fluid supply line (8) is selectively fluidly connectable to the return line (7) of the blood circuit (17).

In a 36^{th} aspect according to the previous aspect, the post infusion tract (58) is connected to a bubble trap (19) placed on the blood return line (7).

In a 37^{th} aspect according to any one of the previous two aspects, a third tract (69) of the infusion line (63) and the post infusion tract (58) of the fluid supply line (8) share a common terminal line tract.

In a 38^{th} aspect according to any one of the previous three aspects, the post infusion tract (58) of the fluid supply line (8) is fluidly connected to the second site (32).

In a 39^{th} aspect according to any one of the previous five aspects, the fluid supply line (8) includes an intake branch (57) extending up to the filtration unit (2).

In a 40^{th} aspect according to any one of the previous six aspects, the disposable set further comprises a pressure detecting site (60a) arranged:
- on the effluent fluid line (13) between the filtration unit (2) and the effluent pump tract (13p), and/or
- on the supply line (8) between the supply pump (25) and the filtration unit (2), optionally on intake branch (57);
in particular said pressure detecting site (60a) being a pressure pod.

In a 41^{st} aspect according to any one of the previous seven aspects, the fluid circuit (16) comprises a dialysis fluid source (14) connected to the supply line (8), the dialysis fluid source (14) including a bag housing a dialysis fluid.

In a 42^{nd} aspect according to any one of the previous aspects, the disposable set further comprises an ion replacement container (73) and an ion replacement fluid line (74) connected to the ion replacement container (73) and to the blood return line (7), optionally at an access site (18) placed downstream the second site (32).

In a 43^{rd} aspect according to any one of the previous aspects, the disposable set does not comprise a one-way valve at the first site (31) and/or at the second site (32).

In a 44^{th} aspect according to any one of the previous aspects, the loop recirculation circuit (11) comprises a bubble trap (19).

In a 44^{th} bis aspect according to any one of the previous aspects, the disposable set further comprises a blood warmer disposable unit, such as a flexible bag or a rigid container, having a blood flow path with an inlet and an outlet for the fluid, the blood warmer disposable unit being fluidly connected the blood circuit (17) upstream the second site (32), in particular the inlet of the blood warmer disposable unit being fluidly connected to an upstream segment of the blood return line (7) and the outlet of the blood warmer disposable unit being fluidly connected to a downstream segment of the blood return line (7), both fluid connections being upstream the second site (32).

In a 44^{th} ter aspect according to aspect 44 bis, the blood warmer disposable unit is included in the loop recirculation circuit (11). In this way blood in the warmer bag is kept in motion during the recirculation procedure.

In a 44^{th} quater aspect according to aspect 44 bis or 44 ter, the blood warmer disposable unit is placed upstream the bubble trap (19).

In a 45^{th} aspect according to any one of the previous aspects, the blood pump tract (6p) is configured to be engaged to a peristaltic blood pump (21).

In a 46^{th} aspect according to any one of the previous aspects when depending on aspect 16, the infusion pump tract (65p) of the infusion line (63) is configured to be engaged by a peristaltic infusion pump (65).

In a 47^{th} aspect according to any one of the previous aspects, the disposable set is engageable to an extracorporeal blood treatment apparatus, such as a CRRT apparatus, a hemodialysis (HD) apparatus, an ultrafiltration (UF) apparatus, a haemofiltration (HF) apparatus, a haemodiafiltration (HDF) apparatus configured for performing a haemodiafiltration treatment, an adsorption apparatus, a carbon dioxide removal (ECCO2R) apparatus, a therapeutic plasma exchange (TPE) or a blood oxygenation apparatus.

In a 48^{th} aspect according to any one of the previous aspects:
- the first site (31) is located between the second end (6b) of the blood withdrawal line (6) and the filtration unit (2), in particular the first site (31) is not coincident with the second end (6b) of the blood withdrawal line (6), and
- the second site (32) is located between the filtration unit (2) and the second end (7b) of the blood return line (7), in particular the second site (32) is not coincident with the second end (7b) of the blood return line (7).

In a 49^{th} aspect according to any one of the previous aspects:
- the first site (31) is distinct from the second end (6b) of the blood withdrawal line (6); and
- the second site (32) is distinct from the second end (7b) of the blood return line (7).

In a 50^{th} aspect according to any one of the previous aspects, the loop recirculation circuit (11) does not include the second end (6b) of the blood withdrawal line (6) and the second end (7b) of the blood return line (7).

In a 51^{st} aspect according to any one of the previous aspects, the apparatus further comprises a safety valve (27) placed on the blood withdrawal line (6) between the second end (6b) of the blood withdrawal line (6) and the first site (31).

In a 52^{nd} aspect according to any one of the previous aspects, the apparatus further comprises a safety valve (20) placed on the blood return line (7) between the second site (32) and the second end (7b) of the blood return line (7).

In a 53^{rd} aspect according to any one of the previous aspects, the disposable set further comprises the loop recirculation circuit (11) that is a close circuit.

Below additional aspects 54 to 73bis refers to the apparatus for extracorporeal blood treatment according to previous aspects 1 and 4; however, the relevant features disclosed therein may be combined with the disposable set features of the preceding depending aspects as well when combined with the apparatus.

In a 54^{th} aspect according to any one of the previous aspects, the control unit (12) is configured to perform a recirculation procedure comprising the steps of:
- defining the loop recirculation circuit (11) by at least:
   ➢ commanding the one or more intercepting elements (20, 27, 59, 70) to put in fluid communication, the first site (31) with the second site (32) through the recirculation portion (66) of the infusion line (63);
- allowing the blood pump (21) to determine blood flow at least within the loop recirculation circuit (11) of the blood circuit (17) of the disposable set.

Safety valve/s (20 and/or 27) on the blood circuit 17 may be left open for example in case of complete obstruction of the patient vascular access.

Further, safety valve/s 20, 27 on the blood circuit 17 may also be left open in case of partial access obstruction scenario; then the control unit (12) still controls the blood pump (21) to determine blood flow within the loop recirculation circuit (11) of the blood circuit (17) of the disposable set, but additionally a blood circulation is maintained in the blood circuit section between second site (32) and the patient and between the patient and the first site (31). Indeed, in the latter scenario, where the obstruction is not 'full/total', a small blood flow 'q' could be taken from the catheter at the second end (6b) and returned to second end (7b), while a recirculation flow 'Q_{b}-q' is present in the infusion line branches, namely second and third tracts (68 and 69).

In a 54^{th} aspect bis, according to any one of the previous aspects, the control unit (12) is configured to perform a recirculation procedure comprising the steps of defining the loop recirculation circuit (11) by at least:
➢ commanding the one or more intercepting elements (20, 27, 59, 70) to prevent fluid passage either or both in the blood withdrawal line (6) between the second end (6b) of the blood withdrawal line (6) and the first site (31) and in the blood return line (7) between the second site (32) and the second end (7b) of the blood return line (7).

In a 55^{th} aspect according to any one of the previous aspects, the one or more intercepting elements (20, 27, 59, 70) comprise an intercepting element (70) acting on the infusion line (63) and movable between:
- a recirculation position, wherein the intercepting element (70) is configured to allow fluid communication between the first site (31) and the second site (32) through the recirculation portion (63) of the infusion line (63);
- a lock position, wherein the intercepting element (70) is configured to prevent fluid communication between the first site (31) and the second site (32) through the recirculation portion (63) of the infusion line (63);
and wherein, before starting the recirculation procedure, the control unit (12) is configured to switch the intercepting element (70) to the recirculation position and to keep the intercepting element (70) in the recirculation position during the recirculation procedure.

In a 55^{th} bis aspect according to the previous aspect, the control unit (12) is not configured to stop the blood pump (21) before starting the recirculation procedure, eventually the control unit (12) is configured to change a blood pump speed before starting the recirculation procedure.

In a 56^{th} aspect according to any one of the previous two aspects, in the lock position, the control unit (12) is configured to command the intercepting element (70) to prevent fluid passage towards either or both the second end (63b) and the third end (63c) of the infusion line (63).

In a 57^{th} aspect according to any one of the previous aspects, the one or more intercepting elements (20, 27, 59, 70) comprise at least one of:
- a first safety valve (20) active on the blood withdrawal line (6) and arranged between the second end (6b) of the blood withdrawal line (6) and the first site (31), the first safety valve (20) being arranged closer to the patient than the first site (31), and
- a second safety valve (27) active on the blood return line (7), and arranged between the second end (7b) of the blood return line (7) and the second site (32), in particular the second safety valve (27) being arranged closer to the patient than the second site (32);
wherein the first safety valve (20) and/or the second safety valve (27) are movable between:
- an open position, wherein blood passage, respectively through the blood withdrawal line (6) and the blood return line (7), is allowed; and
- a close position, wherein blood passage, respectively through the blood withdrawal line (6) and the blood return line (7), is prevented,

In a 57^{th} bis aspect according to the previous aspect, the control unit (12) is configured to keep the first safety valve (20) and/or the second safety valve (27) to the open position during the recirculation procedure.

In a 57^{th} ter aspect according to the previous aspect 57, wherein, in the recirculation procedure, the control unit (12) is configured to move the first safety valve (20) and/or the second safety valve (27) to the close position to prevent fluid passage.

In a 58^{th} aspect according to any one of the previous aspects, one or more pumps (21, 25, 26, 54, 65, 75) comprises an infusion pump (65) active on the infusion pump tract (63p) of the infusion line, wherein, in the recirculation procedure, the control unit (12) is configured to keep said infusion pump (65) inactive, in particular the infusion pump (65) being an occlusive pump, such as a peristaltic pump.

In a 58^{th} bis aspect according to any one of the previous aspects, the control unit (12) is configured to stop the blood pump (21) before starting the recirculation procedure.

In a 59^{th} aspect according to the previous aspect, the infusion pump (65) active on the infusion line (63) is arranged between the first end (63a) of the infusion line (63) and the branch site (67) upstream the branch site.

In a 59^{th} bis aspect according to any one of the previous aspects, after stopping the blood pump (21) and before starting the recirculation procedure, the control unit (12) is configured to raise a blood level in a bubble trap (19) particularly to avoid recirculating air bubbles.

In a 60^{th} aspect according to any one of the previous aspects, during the recirculation procedure, the control unit (12) is configured to stop any ultrafiltration of fluids from a primary chamber (3) towards a secondary chamber (4) of the filtration unit (2).

In a 61^{st} aspect according to any one of the previous aspects, during the recirculation procedure, the control unit (12) is configured to keep the patient (P) fluidly isolated from the loop recirculation circuit (11).

In a 61^{st} aspect according to any one of the previous aspects 1 to 60, during the recirculation procedure, the control unit (12) is not configured to keep the patient (P) fluidly isolated from the loop recirculation circuit (11).

In a 62^{nd} aspect according to any one of the previous aspects, during the recirculation procedure, the control unit (12) is configured to recirculate blood in the loop recirculation circuit (11) to prevent blood from clotting.

In a 63^{rd} aspect according to any one of the previous aspects, during the recirculation procedure, the control unit (12) is configured to interrupt the extracorporeal blood treatment.

In a 64^{th} aspect according to any one of the previous aspects, the loop recirculation circuit (11) defines an optionally close inner volume wherein the control unit (12) is configured to recirculate blood during the recirculation procedure.

In a 65^{th} aspect according to any one of the previous aspects, the disposable set further comprises a pre-blood-pump infusion line (51) extending from a first end (51a) for connection to a second fluid source (10), and a second end (51b) fluidly connected to the blood withdrawal line (6), said one or more pumps (21, 25, 26, 54, 65, 75) comprising a PBP infusion pump (54) acting on the pre-blood-pump infusion line (51).

In a 66^{th} aspect according to the previous aspect, during the recirculation procedure, the control unit (12) is configured to keep the PBP infusion pump (54) inactive, in particular the PBP infusion pump (54) being an occlusive pump, such as a peristaltic pump.

In a 66^{th} bis aspect according to any one of the previous aspects, the disposable set comprises a blood warmer disposable unit having a blood flow path with an inlet and an outlet, the blood warmer disposable unit being fluidly connected the blood circuit (17) upstream the second site (32), in particular the inlet of the blood warmer disposable unit being fluidly connected to the blood return line (7) and the outlet of the blood warmer disposable unit being fluidly connected to the blood return line (7) upstream the second site (32),
during the recirculation procedure, the control unit (12) being configured to recirculate the blood contained in the blood warmer bag.

In a 66^{th} ter aspect according to aspect 66 bis, wherein, during the recirculation procedure, the control unit (12) is configured to:
- stop heating the blood warmer bag; or
- adjust a set point temperature of a warmer unit, in particular as a function of a patient actual blood temperature or of a patient desired blood temperature or as a default setting.

In a 67^{th} aspect according to any one of the previous aspects, the control unit (12) is configured to determine an alarm situation for the apparatus and to start, preferably automatically, the recirculation procedure once the alarm situation is detected.

In a 68^{th} aspect according to the previous aspect, upon alarm situation is detected, the control unit (12) is configured to stop the blood pump (21) and, optionally to close a second safety valve (20) on the blood return line (7) and/or a first safety valve (27) on the blood withdrawal line (6).

In a 69^{th} aspect according to any one of the previous two aspects, upon alarm situation is detected, the control unit (12) is configured to check whether an infusion fluid source (64) is available to the infusion line (63) and to provide an alarm and/or to abort the recirculation procedure in case the infusion fluid source (64) is not available.

In a 70^{th} aspect according to any one of the previous aspects, during the recirculation procedure, the control unit (12) is configured to run the blood pump (21) at a speed different than a speed of the blood pump (21) during the extracorporeal blood treatment.

In a 70^{th} bis aspect according to any one of the previous aspects, during the recirculation procedure, the control unit (12) is configured to run the blood pump (21) in a same direction as during the extracorporeal blood treatment. In a 70^{th} ter aspect according to any one of the previous aspects, wherein the control unit (12) is configured to end the recirculation procedure, once a user has cleared an alarm triggering the recirculation procedure.

In a 70^{th} quater aspect according to any one of the previous aspects, wherein the control unit (12) is configured end the recirculation procedure in case a lapsed recirculation time is higher than a pre-set recirculation time, optionally wherein the pre-set recirculation time is a function of the anticoagulation mode during extracorporeal blood treatment.

In a 70^{th} quinquies aspect according to any one of the previous aspects, wherein the control unit (12) is configured to request confirmation to a user, e.g., via a user interface, to resume the treatment once the recirculation procedure is ended.

Alternatively, the user may select to stop the treatment (e.g., for changing the disposable set).

In a 71^{st} aspect according to any one of the previous aspects, to end the recirculation procedure, the control unit (12) is configured to command the one or more intercepting elements (20, 27, 59, 70) to prevent fluid communication between the first site (31) and the second site (32) through the recirculation portion (66) of the infusion line (63).

In a 71^{st} bis aspect according to any one of the previous aspects, to resume the extracorporeal blood treatment after recirculation, the control unit (12) is configured to check a blood level in a bubble trap (19) and, if necessary, to provide an adjustment, e.g., via an air pump (47), for example according to a pressure level during recirculation versus a return pressure operating point prior to the recirculation procedure.

In a 72^{nd} aspect according to the previous aspect 71, after preventing fluid communication between the first site (31) and the second site (32) through the recirculation portion (66), the control unit (12) is configured to ramp-up or decrease the blood pump (21) to a set flow rate for the extracorporeal blood treatment.

In a 73^{rd} aspect according to any one of the previous two aspects, wherein the control unit (12) is configured to:
a. move an intercepting element (70) acting on the infusion line (63) to a lock position allowing fluid flow in a second tract (68) extending from the branch site (67) up to the second end (63b) of the infusion line (63) and blocking a fluid flow in a third tract (69) extending from the branch site (67) up to the third end (63c) of the infusion line (63) to prevent fluid communication between the first site (31) and the second site (32) through the recirculation portion (66);
b. rinse the blood in the second tract (68) with a fluid from the infusion line (63).

In a 73^{rd} bis aspect according to any one of the previous 3 aspects, wherein the control unit is configured to:
a. move an intercepting element (70) acting on the infusion line (63) to a lock position allowing fluid flow in a third tract (69) extending from the branch site (67) up to the third end (63c) of the infusion line (63) and blocking a fluid flow in a second tract (68) extending from the branch site (67) up to the second end (63b) of the infusion line (63) to prevent fluid communication between the first site (31) and the second site (32) through the recirculation portion (66);
b. rinse the blood in the third tract (69) with a fluid from the infusion line (63).

Below additional aspects 74 to 89 refers to the method of recirculating blood in an extracorporeal blood treatment apparatus according to previous aspect 3; however, the relevant features disclosed therein may be combined with the disposable set and/or the apparatus features of the preceding dependent aspects as well when combined with the method aspect.

In a 74^{th} aspect according to any one of the previous aspects when combined with method aspect 3, the method further comprises:
- to define the loop recirculation circuit (11) including:
   ∘ to command the one or more intercepting elements (20, 27, 59, 70) to put in fluid communication, the first site (31) with the second site (32) through the recirculation portion (66) of the infusion line (63);
- to allow the blood pump (21) to determine blood flow within the loop recirculation circuit (11) of the blood circuit (17) of the disposable set.

In a 74^{th} bis aspect according to the previous aspect, when combined with method aspect 3, to define the loop recirculation circuit (11) includes to command the one or more intercepting elements (20, 27, 59, 70) to prevent fluid passage either or both in the blood withdrawal line (6) between the second end (6b) of the blood withdrawal line (6) and the first site (31) and in the blood return line (7) between the second site (32) and the second end (7b) of the blood return line (7).

In a 74^{th} ter aspect according to the previous aspect 74, to define the loop recirculation circuit (11) includes to command the one or more intercepting elements (20, 27, 59, 70) to allow fluid passage either or both in the blood withdrawal line (6) between the second end (6b) of the blood withdrawal line (6) and the first site (31) and in the blood return line (7) between the second site (32) and the second end (7b) of the blood return line (7).

In a 74^{th} quater aspect according to the previous aspect, the method comprises controlling the blood pump (21) to determine blood flow within the loop recirculation circuit (11) of the blood circuit (17) of the disposable set, and maintaining additionally a blood circulation in the blood circuit section between second site (32) and the patient and between the patient and the first site (31), in particular wherein blood is withdrawn at the second end (6b) of the blood withdrawal line (6) and returned to second end (7b) of the blood return line (7), a blood flow (q) being generated in the blood circuit (17) upstream the first site (31) and downstream the second site (32), a recirculation flow (Q_{b}-q) being present in the recirculation portion (66) of the infusion line (63), namely in the second and the third tracts (68 and 69), and in a remaining portion of the blood circuit (17) comprising the blood pump tract (6p) and the filtration unit (2).

In a 75^{th} aspect according to any one of the previous aspects 74 to 74 quater or to aspect 3, the one or more intercepting elements (20, 27, 59, 70) comprise an intercepting element (70) acting on the infusion line (63) and movable between:
- a recirculation position, wherein the intercepting element (70) is configured to allow fluid communication between the first site (31) and the second site (32) through the recirculation portion (63) of the infusion line (63);
- a lock position, wherein the intercepting element (70) is configured to prevent fluid communication between the first site (31) and the second site (32) through the recirculation portion (63) of the infusion line (63);
the method comprising, before starting the recirculation procedure, to switch the intercepting element (70) to the recirculation position, and to keep the intercepting element (70) in the recirculation position during the recirculation procedure.

In a 76^{th} aspect according to any one of the previous two aspects or to aspect 3, the one or more intercepting elements (20, 27, 59, 70) comprise at least one of:
- a first safety valve (20) active on the blood withdrawal line (6) and arranged between the second end (6b) of the blood withdrawal line (6) and the first site (31), and
- a second safety valve (27) active on the blood return line (7), and arranged between the second end (7b) of the blood return line (7) and the second site (32);

wherein the first safety valve (20) and/or the second safety valve (27) are movable between:
   - an open position, wherein blood passage, respectively through the blood withdrawal line (6) and the blood return line (7), is allowed; and
   - a close position, wherein blood passage, respectively through the blood withdrawal line (6) and the blood return line (7), is prevented,
the method comprises alternatively:
   - before the recirculation procedure, to move the first safety valve (20) and/or the second safety valve (27) to the close position to prevent fluid passage and to keep the first safety valve (20) and/or the second safety valve (27) to the close position during the recirculation procedure, or
   - not to switch the first safety valve (20) and/or the second safety valve (27) to the close position and to keep the first safety valve (20) and/or the second safety valve (27) to the open position during the recirculation procedure.

In a 77^{th} aspect according to any one of the previous aspects 74 to 76 or to aspect 3, the extracorporeal blood treatment apparatus further comprises an infusion pump (65) engaging a first infusion tract (72) of the infusion line (63), the method further comprising the steps of:
- stopping or keeping inactive the infusion pump (65);
- allowing fluid circulation in the recirculation portion (66) of the infusion line (63) downstream the branch site (67) and between the second site (32) and the first site (31); and
- after stopping the infusion pump (65) and allowing circulation, activating the blood pump (21), if previously stopped.

In a 78^{th} aspect according to any one of the previous aspects 74 to 77 or to aspect 3, the method further comprises, during recirculation, stopping any ultrafiltration of fluids from a primary chamber (3) towards a secondary chamber (4) of the filtration unit (2).

In a 79^{th} aspect according to any one of the previous aspects 74 to 78 or to aspect 3, the method further comprises, during recirculation, keeping the patient (P) fluidly isolated from the loop recirculation circuit (11).

In an 80^{th} aspect according to any one of the previous aspects 74 to 79 or to aspect 3, the method further comprises, during recirculation, not keeping the patient (P) fluidly isolated from the loop recirculation circuit (11).

In an 81^{st} aspect according to any one of the previous aspects 74 to 79 or to aspect 3, the method further comprises, during recirculation, recirculating blood in the loop recirculation circuit (11) to prevent blood from clotting.

In an 82^{nd} aspect according to any one of the previous aspects 74 to 81 or to aspect 3, the method further comprises ending the recirculation procedure, once a user has cleared an alarm triggering the recirculation procedure.

In an 83^{rd} aspect according to any one of the previous aspects 74 to 82 or to aspect 3, the method further comprises ending the recirculation procedure in case a lapsed recirculation time is higher than a pre-set recirculation time, optionally wherein the pre-set recirculation time is a function of the anticoagulation mode during extracorporeal blood treatment.

In an 84^{th} aspect according to any one of the previous aspects 74 to 83 or to aspect 3, the method further comprises requesting confirmation to a user, e.g., via a user interface, to resume the treatment once the recirculation procedure is ended and/or requesting confirmation to a user, e.g., via a user interface, to stop the treatment.

In an 85^{th} aspect according to any one of the previous aspects 74 to 84 or to aspect 3, the method further comprises, to resume the extracorporeal blood treatment after recirculation, checking a blood level in a bubble trap (19) and, if necessary, provide an adjustment, e.g., via an air pump (47), for example according to a pressure level during recirculation versus a return pressure operating point prior to the recirculation procedure.

In an 86^{th} aspect according to any one of the previous aspects 74 to 85, or to aspect 3, the method comprises, to end the recirculation procedure, commanding the one or more intercepting elements (20, 27, 59, 70) to prevent fluid communication between the first site (31) and the second site (32) through the recirculation portion (66) of the infusion line (63).

In an 87^{th} aspect according to aspect 86, the method further comprises, after preventing fluid communication between the first site (31) and the second site (32) through the recirculation portion (66), ramping-up or decreasing the blood pump (21) to a set flow rate for the extracorporeal blood treatment.

In an 88^{th} aspect according to any one of the previous two aspects, the method comprises:
a. moving an intercepting element (70) acting on the infusion line (63) to a lock position allowing fluid flow in a second tract (68) extending from the branch site (67) up to the second end (63b) of the infusion line (63) and blocking a fluid flow in a third tract (69) extending from the branch site (67) up to the third end (63c) of the infusion line (63) to prevent fluid communication between the first site (31) and the second site (32) through the recirculation portion (66);
b. rinsing the blood in the second tract (68) with a fluid from the infusion line (63).

In an 89^{th} aspect according to any one of the previous three aspects, the method comprises:
a. moving an intercepting element (70) acting on the infusion line (63) to a lock position allowing fluid flow in a third tract (69) extending from the branch site (67) up to the third end (63c) of the infusion line (63) and blocking a fluid flow in a second tract (68) extending from the branch site (67) up to the second end (63b) of the infusion line (63) to prevent fluid communication between the first site (31) and the second site (32) through the recirculation portion (66);
b. rinsing the blood in the third tract (69) with a fluid from the infusion line (63).

### DRAWINGS

Some embodiments and some aspects of the disposable set and apparatus will be described below with reference to the attached drawings, provided for illustrative purposes only, wherein:
- Figure 1 is a schematic view of the fluid lines of a disposable set coupled to pumps, sensors and actuators of a related apparatus according to an embodiment of the present invention;
- Figure 1a represents the scheme of figure 1 with a recirculation loop highlighted by a thicker line;
- Figure 2 is a schematic view of the fluid lines of a disposable set coupled to pumps, sensors and actuators of a related apparatus according to the following description;
- Figure 2a represents the scheme of figure 2 with the recirculation loop highlighted by a thicker line;
- Figure 3 is a representation of a disposable set coupled to pumps, sensors and actuators of a related apparatus according to the following description;
- Figure 3a mirrors the scheme of figure 3, wherein the recirculation loop is schematically highlighted by a thicker line;
- Figure 4 is a representation of a further embodiment of a disposable set mounted on a related treatment apparatus according to a second embodiment of the present invention;
- Figure 4a mirrors the scheme of figure 4, wherein the recirculation loop is schematically highlighted by a thicker line;
- Figure 5 is a representation of an alternative embodiment of a disposable set mounted on a related treatment apparatus according to a third embodiment of the present invention;
- Figure 5a mirrors the scheme of figure 5, wherein the recirculation loop is schematically highlighted by a thicker line.

### DEFINITIONS

In this detailed description, corresponding parts illustrated in the various figures are indicated with the same numerical references. The figures may illustrate the features by means of non-scale representations; therefore, parts and components illustrated in the figures may relate exclusively to schematic representations.

### Upstream and downstream

The terms upstream and downstream refer to a direction or trajectory of advancement of the fluid or blood configured to flow within a line of the disposable set during a usual blood treatment procedure, wherein blood is withdrawn from the patient at a first access site, circulated into the blood withdrawal line, the filtration unit and the blood return line before being re-injected into the patient at a second access site. Infusion lines directs the respective fluid into the extracorporeal blood circuit and the fresh dialysis fluid is fed to the filtration unit (countercurrent to the blood) and removed via an effluent line towards a drain.

### DETAILED DESCRIPTION

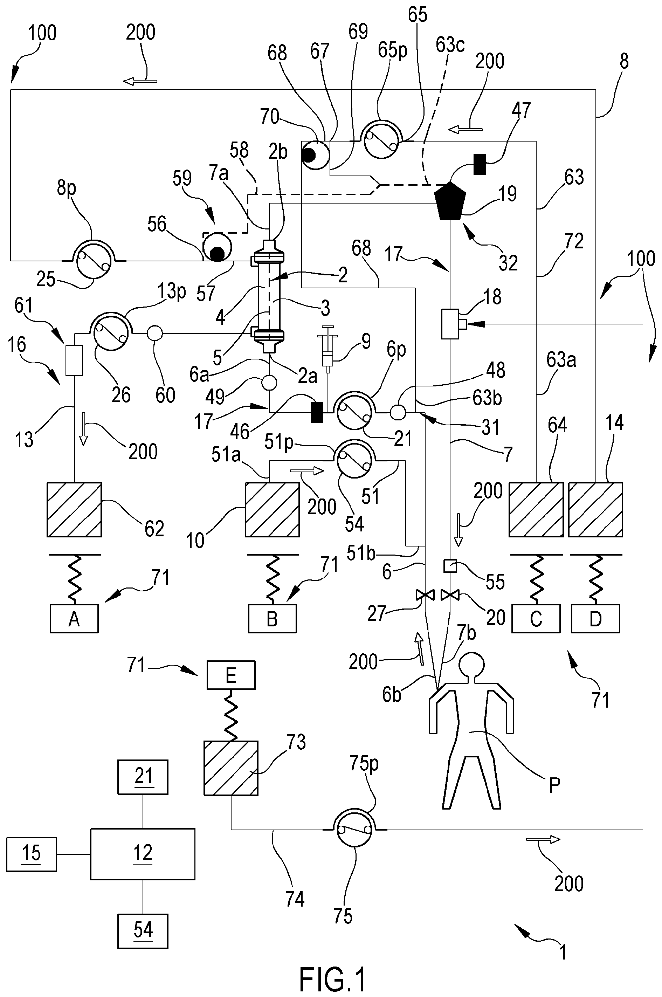

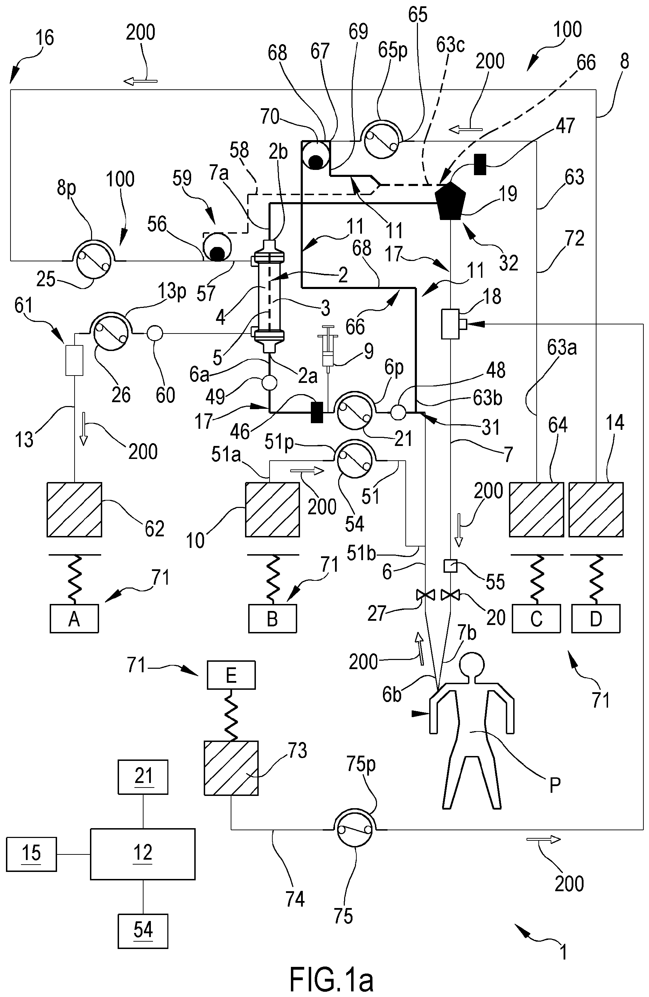

With reference to figure 1, reference number 1 generally refers to the extracorporeal blood treatment apparatus, particularly, but not exclusively, for intensive care therapies. The extracorporeal blood treatment apparatus of the present invention may perform any dialysis treatments such as hemodialysis (HD), hemofiltration (HF), hemodiafiltration (HDF), or ultrafiltration (UF), as well as (C)RRT treatments; the apparatus may alternatively/additionally be configured for ECCO2R or oxygenation treatments, as well as for adsorption, liver treatments, hemoperfusion, or plasmapheresis. The extracorporeal blood treatment apparatus 1 is designed to receive a disposable set 100 as claimed in the present application and below described.

The apparatus according to figure 1 is particularly (but not exclusively) designed for Renal Replacement Therapies (RRT) and particularly for Continuous Renal Replacement Therapies (CRRT). CRRT apparatuses are configured to deliver very specific treatments designed for patients versing in acute states of illness and who have temporarily lost their kidney function. In this respect, CRRT systems may be structurally and/or operationally different from extracorporeal blood treatment systems designed for chronic patient care. CRRT systems need to exhibit specific technical features enabling the system to:
- allow setting of a weight loss rate,
- continuously remove excess water in accordance with a set weight loss rate,
- operate continuously at comparably low flow rates compatible with CRRT, and
- balance ion equilibrium by means of proper dialysis being performed and/or by means of substitution fluid continuously being delivered at controlled flow rates.

Finally, in order to prepare a CRRT apparatus as soon as possible given the acute situation of a patient requiring a treatment and given the absence of advance notice of the emergency situation requiring treatment, the CRRT machine is dressed using an integrated disposable set 100, wherein all the lines and the filtration unit are grouped together and already properly connected in the disposable set. Further, all the fluids are normally (but not necessarily) contained in pre-packaged bags (dialysis fluid or replacement fluids in bags of e.g., 2, 5 or 10 litres each) or pre-packaged syringes (heparin and/or concentrated calcium replacement solution).

Notwithstanding the specifically described embodiments are referred to extracorporeal blood treatment apparatus and disposable sets for acute therapy, the present invention is not limited to such field and may also be implemented and used in apparatus for the treatment of chronic patients.

Reference number 100 is directed to a disposable set for the extracorporeal blood treatment apparatus. The disposable set 100, schematically shown in figures 1-5, comprises blood lines and fluid lines respectively configured to be engaged to the treatment apparatus 1, the latter comprising for example peristaltic pumps to promote fluid and blood flows.

The disposable set 100 comprises at least one filtration unit 2, which is configured to treat the blood withdrawn from the patient. The filtration unit 2 may be any kind of filter to treat blood, such as a filter for performing haemodialysis (HD), ultrafiltration (UF), haemofiltration (HF) and haemodiafiltration (HDF), for hemoperfusion, a plasmafilter, an adsorber, etc...

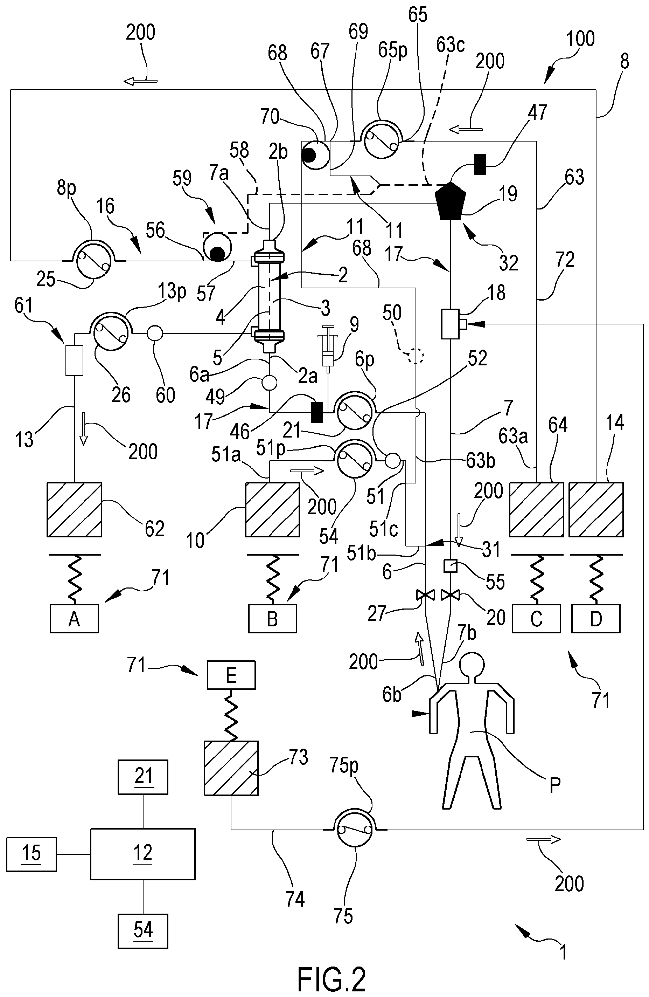

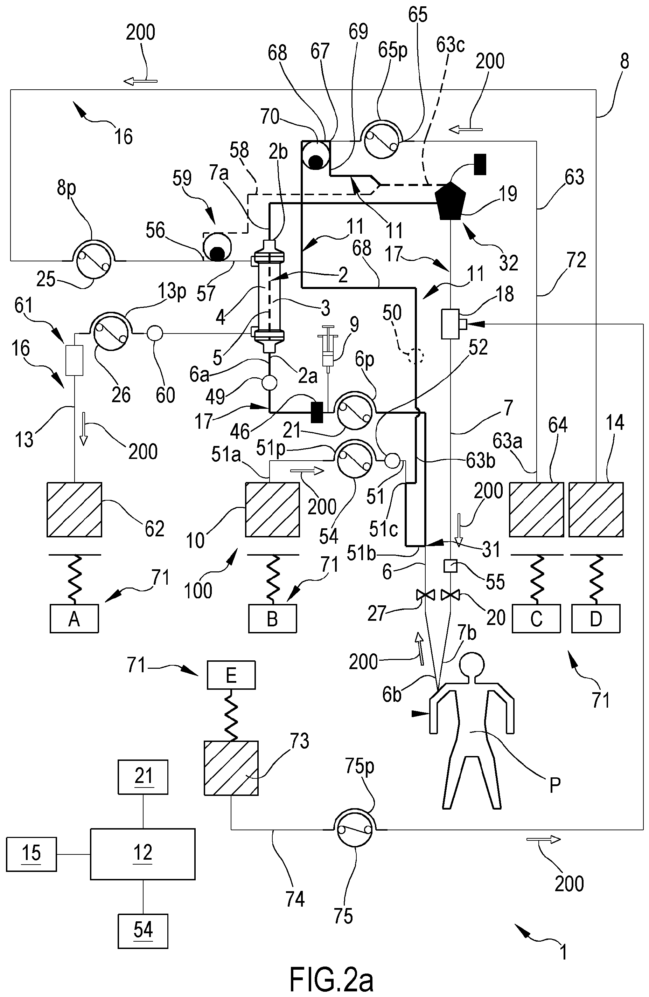

As shown in figures 1 and 2, in certain embodiments, the filtration unit 2 has a primary chamber 3 and a secondary chamber 4 separated by a semi-permeable membrane 5, wherein the primary chamber 3 receives blood withdrawn from the patient, while the secondary chamber 4 receives waste products filtered out from the blood and discharges them through a fluid outlet connected to an effluent fluid line 13. Depending upon the treatment, the membrane 5 of the filtration unit 2 may be selected to have different properties and performances.

A blood circuit 17 comprises a blood withdrawal line 6 extending between a first end 6a connected to a blood inlet 2a of the primary chamber 3 of the filtration unit 2, and a second end 6b for connection to a patient P. The blood withdrawal line 6 is configured to receive blood from the patient P and to allow blood circulation along a withdrawal direction 200 from the second end 6b towards the first end 6a of the blood withdrawal line 6 and into the filtration unit 2. The blood circuit 17 further comprises a blood return line 7 extending between a first end 7a connected to a blood outlet 2b of the primary chamber 3 of the filtration unit 2, and a second end 7b for connection to the patient P. The blood return line 7 is configured to receive (treated) blood from the blood outlet 2b of the filtration unit 2 and to allow blood circulation along a return direction from the first end 7a towards the second end 7b of the blood return line 7. The blood withdrawal line 6 and the blood return line 7 are usually made of a flexible material, for example PVC or a plastic based material, and may also be transparent to allow an operator to see the blood flowing within the lines. The blood circuit 17 further comprises a pump tract 6p configured to engage a blood pump 21 of the extracorporeal blood apparatus to promote blood flow in the blood withdrawal line 6 and in the blood return line 7. The pump tract 6p may be arranged on the blood withdrawal line 6 or on the blood return line 7. Alternatively, the blood circuit 17 may comprise two blood pumps, wherein a first blood pump is engaged to the blood withdrawal line 6, and a second blood pump is engaged to the blood return line 7 (this embodiment is not shown). Please note that pump symbols in the drawings do not necessarily indicate the direction of the flow inside the respective pump tract. Generally, each and every (e.g., peristaltic) pump may be controlled in one direction and in the opposite direction, too, depending on the apparatus and therapy needs.

As shown in the embodiments of figures 1 to 5, the blood withdrawal line 6 includes the pump tract 6p that is engaged by the blood pump 21 of the extracorporeal blood treatment apparatus. The blood pump 21 is configured to determine a blood flow in a direction 200 from the second end 6b of the withdrawal line towards the filtration unit 2 and then to back to the blood return line 7.

In a first example and as represented in the drawings, the blood pump tract 6p may be a portion of the blood withdrawal line 6 itself, which is interposed between the first end 6a and the second end 6b of the blood withdrawal line 6. The blood pump tract 6p may be different with respect to the other line tracts of the blood withdrawal line 6 in terms of dimension (e.g. passage section and/or wall thickness) and/or material. In particular the blood pump tract 6p may have an external dimension, i.e. an external diameter, larger than an external dimension, i.e. an external diameter, of the first and/or second tracts of the blood withdrawal line 6. In addition or alternatively, the blood pump tract 6p may have an internal dimension, i.e. an inner diameter, defining the fluid passage of the blood pump tract 6p, which is larger than an internal dimension, i.e. an internal diameter, of the first and/or second tracts of the blood withdrawal line. The blood pump tract 6p may be a separate and different tube segment with respect to the other line tracts of the blood withdrawal line 6, wherein the blood pump tract 6p is engaged/coupled to the other tracts by gluing or welding process during a manufacturing process of the disposable set 100 (and/or a suitable connector may be interposed between the end of the pump tract and the other tube portions of the withdrawal line to join the respective ends). The blood pump tract 6p may also be curved (e.g., a Ω shape) and/or may have elastic properties different from the properties of the first and the other line tracts of the blood withdrawal line 6: for example the blood pump tract 6p may be more flexible than the other line tracts of the blood withdrawal line 6 e.g., to withstand the fatigue stresses caused by the peristaltic pump 21 of the blood treatment apparatus. In an alternative, the blood pump tract 6p may be a disposable pump header to be connected to the pump body e.g., by magnetic coupling. The blood pump 21 may be a centrifuge pump. This is for example the case of a centrifuge pump that may be used in ECMO circuit

During treatment, the blood withdrawal line 6 and the blood return line 7 are connected directly or indirectly to the blood cardiovascular system of the patient through an access device such as a needle, a catheter, or a graft. Normally, in a renal replacement treatment the blood withdrawal line 6 and the blood return line 7 are directly connected to the blood cardiovascular system of the patient; however, in certain situations, such as when the patient is subject to a cardiopulmonary by-pass and therefore already connected to an ECMO machine, the blood withdrawal line 6 and the blood return line 7 may be connected to the ECMO extracorporeal blood lines and consequently indirectly connected to the blood cardiovascular system of the patient (via the ECMO blood lines).

The apparatus 1 comprises a blood pump 21 for controlling the blood flow in the blood circuit 17. The blood pump 21 is generally a peristaltic pump acting either on the blood withdrawal line 6 (as shown e.g. in figures 1-5) and/or on the blood return line 7 (embodiment not shown). An operator may enter a set value for the blood flow rate through a user interface 15 and a control unit 12, during treatment, is configured to control the blood pump based on the set blood flow rate.

Blood is withdrawn from the patient at a vascular access and flows through the blood withdrawal line 6; after passing through the primary chamber 3 of the filtration unit 2, where the suitable exchanges of substances, molecules and fluids occur through the semipermeable membrane 5, the treated blood enters the blood return line 7, first passing through the air separator, commonly known as "*bubble trap*", 19 designed to ensure removal of air bubbles present in the blood. The treated blood getting out of the bubble trap 19, before being returned to the patient P passes through an air bubble sensor 55 verifying the absence of air bubbles within the treated blood that has to be reintroduced in the patient's blood vascular circuit at the venous access. Downstream the air bubble sensor 55, a safety valve 20 (or venous clamp) is provided: the safety valve 20, in case of alarm requiring stop of the blood pump and patient isolation, blocks the blood flow towards the patient. For example, should the bubble sensor 55 detect the presence of air/gas in the blood flow, the control unit 12 controls the safety valve 20 to the close position to prevent air to be infused into the patient.

A corresponding safety valve 27 (or arterial clamp) may be present on the blood withdrawal line 6 close to the patient vascular access to fully isolate the patient from the extracorporeal blood circuit in case of need.

The safety valve 27 of the blood withdrawal line 6 and the safety valve 20 of the blood return line 7 may be arranged both in the close position so that the patient P is fluidly isolated from the blood circuit 17 of the disposable set. The safety valve 27 of the blood withdrawal line 6 and the safety valve 20 of the blood return line 7 may be manually controllable, but are usually automatically controllable by the control unit 12 between the close position and the open position, and vice versa.

The disposable set 100 further comprises an infusion line 63 extending from a first end 63a, for connection to an infusion fluid source 64, and a branch site 67 branching off the infusion line 63 towards a second end 63b and towards a third end 63c. The portion of the infusion line 63 interposed between the second end 63b and the third end 63c of the infusion line 63 defines a recirculation portion 66 of the infusion line, as described in more detail here-after in the present description.

The fluid source 64 is a source of infusion or replacement fluid. In particular, the fluid source 64 may be a plastic bag housing a pre-packaged replacement fluid solution.

The second end 63b of the infusion line 63 is in direct or indirect fluid communication with the blood withdrawal line 6 at a first site 31. A direct fluid communication between the infusion line 63 and the blood withdrawal line 6 means that the infusion line 63 is directly connected to the blood withdrawal line 6 and the fluid exiting the second end 63b of the infusion line 63 enters directly into the blood stream flowing in the blood withdrawal line 6 of the blood circuit 17.

An indirect fluid communication between the infusion line 63 and the blood withdrawal line 6 means that the infusion line 63 is not directly connected to the blood withdrawal line 6, but to another fluid line (e.g., PBP line 51) that is itself directly connected to the blood withdrawal line 6. As a consequence, the infusion fluid exiting the second end 63b of the infusion line 63 enters the other fluid line and then reaches the blood withdrawal line 6 at the injection point of the other line into the blood withdrawal line 6.

As below further explained, an example of direct fluid communication is shown in figures 1 and 1a (and 4, 4a, 5, 5a), while an example of indirect fluid communication is shown in figures 2 and 2a (and 3, 3a).

The first site 31 is located on the blood circuit 17 and is specifically arranged on the blood withdrawal line 6 and is interposed between the blood pump tract 6p and the second end 6b of the blood withdrawal line 6. Even though not essential, the first site 31 may be arranged as close as possible to the patient, so that the remaining portion of the withdrawal line 6 between the first site 31 and the patient is as short as possible. The first site 31 is upstream the blood pump tract 6p with respect to the blood withdrawal direction 200.

As apparent, the first site 31 may or may not coincide with the second end 63b of the infusion line 63. In some embodiments, the first site 31 is coincident with the location where the second end 63b of the infusion line 63 (directly) connects to the blood withdrawal line 6 (e.g., fig. 1); in other embodiments, the first site 31 (which is on the blood withdrawal line 6) may be distinct and spatially separated from the second end 63b of the infusion line 63 (e.g., fig. 2). The first site 31 may comprise a fluid connector, i.e. a three-way fluid connector, including a blood inlet (directly) fluidly connected to the withdrawal line 6 and facing the second end 6b of the blood withdrawal line 6, a blood outlet (directly) fluidly connected to the withdrawal line 6 and facing the first end 6a of the blood withdrawal line 6, in particular facing the pump tract 6p of the blood withdrawal line 6, and an infusion inlet (directly or indirectly) in fluid communication with the second end 63b of the infusion line 63.

The fluid connector may be made by plastic material stiffer than the material of the infusion line 63 and/or of the blood withdrawal line 6. On the contrary, the infusion line 63 is flexible, in particular more flexible than the fluid connector. Analogously, the blood withdrawal line 6 is more flexible than the fluid connector.

Notably, the first site 31 is distinct and spatially separated from the second end 6b of the blood withdrawal line 6. The third 63c end of the infusion line 63 is in direct or indirect fluid communication with the blood return line 7 at a second site 32. The second site 32 is interposed between the blood pump tract 6p and the second end 7b of the blood return line 7. In particular, when the blood pump tract 6p is on the blood withdrawal line 6, the second site 32 is interposed between the blood outlet 2b of the filtration unit 2 and the second end 7b of the return line 7. The second site 32 may comprise a fluid connector, too, i.e. a three-way fluid connector, including a blood inlet (directly) fluidly connected to the return line 7 and facing the first end 7a of the blood return line 7, a blood outlet (directly) fluidly connected to the return line 7 and facing the second end 7b of the blood return line 7, and an infusion inlet (directly or indirectly) in fluid communication with the third end 63c of the infusion line 63. According to the embodiment shown in figures 1 and 2, a bubble trap 19 may be located at the second site 32 arranged on the blood return line 7. The bubble trap 19 includes a blood inlet (directly) fluidly connected to the return line 7 and facing the first end 7a of the blood return line 7, a blood outlet (directly) fluidly connected to the return line 7 and facing the second end 7b of the blood return line 7, and an infusion inlet (directly) fluidly connected to the third end 63c of the infusion line 63. The bubble trap 19 is configured to separate air/gas bubbles from the blood stream, in order to prevent bubbles to flow towards the patient. Notably, the second site 32 is distinct and spatially separated from the second end 7b of the blood return line 7.

As previously mentioned, the infusion line 63 extends from the first end 63a thereof for a first tract 72 up to the branch site 67, where the first tract 72 branches into a second tract 68, extending from the branch site 67 up to the second end 63b of the infusion line 63, and into a third tract 69 extending from the branch site 67 up to the third end 63c of the infusion line 63. A recirculation portion 66 of the infusion line 63 comprises (and may be entirely defined by - in the case of figures 1, 1a, 4, 4a, 5, and 5a) the second tract 68 and the third tract 69 of the infusion line 63.

The infusion line 63 further comprises a respective infusion pump tract 65p, interposed between the first end 63a and the branch site 67 of the infusion line 63; the infusion pump tract 65p of the infusion line 63 is configured to be engaged by a respective infusion pump 65 of the extracorporeal blood treatment apparatus, i.e. an occlusive pump such as a peristaltic pump, configured to cause a fluid flow in a direction from the first end 63a of the infusion line towards the branch site 67. The infusion pump tract 65p may comprise the same technical features previously described and differentiating the blood pump tract 6p from the other line tracts of the blood withdrawal line 6.

Correspondingly, the apparatus 1 includes the infusion pump 65 (in the example a peristaltic pump) engaged to the infusion line 63 and configured to determine, during infusion, the flow rate along the line of the substance housed in the infusion fluid source 64, i.e. replacement fluid.

The extracorporeal blood treatment apparatus 1 may further comprise a selector 70 active on the infusion line 63 at the branch site 67. The selector 70 may be switchable (at least) between:
- a first operating position (pre-infusion), wherein the selector 70 allows fluid communication between the first tract 72 and the second tract 68 of the infusion line 63, and prevents fluid communication between the first tract 72 and the third tract 69 of the infusion line 63;
- a second operating position (post-infusion), wherein the selector 70 prevents fluid communication between the first tract 72 and the second tract 68 of the infusion line 63, and allows fluid communication between the first tract and the third tract 69 of the infusion line 63; and
- a recirculation position (neutral position), wherein the second tract 68 and the third tract 69 of the infusion line 63 are in fluidly communication with each other.

In particular, when the selector 70 is in the recirculation position, both the second tract 68 and the third tract 69 of the infusion line 63 define the recirculation portion 66 of the loop recirculation circuit 11. Furthermore, when the selector 70 is in the recirculation position, the first site 31 is in fluid communication with the second site 32 through the recirculation portion 66. In other terms, in the recirculation position, the selector 70 (which may be a valve clamping either the second tract 68 or the third tract 69 or none of the tubes 68, 69) is not acting on (clamping) any tube portions and is in a neutral and distanced position from the second and third tracts 68, 69.

In the recirculation position, no fluid flows along the first tract 72 of the infusion line 63. For example, the infusion pump 65 may be an occlusive pump and may be inactive thereby preventing any flow; alternatively, a clamp, the same selector 70, or another device may prevent flow in the first tract 72 e.g., by closing it.

Differently, in either the first or the second operating position, fluid flows from the first tract 72 towards the second and/or third tracts 68, 69 and infuse replacement fluid in the blood circuit. In this situation, the cooperation between an active infusion pump 65 and the selector 70 may define the quantity of fluid pre-infused and the quantity of fluid simultaneously post-infused.

Of course, in case only pre- or post-infusion at a time are manageable (i.e., either 100% in pre-infusion or 100% in post- infusion), then the selector 70 prevents fluid communication between the first site 31 and the second site 32 through the recirculation portion 66 of the loop recirculation circuit 11. In particular, in the first operating position, the passage of fluid from the fluid source 64 to the second tract 68 is allowed, while the passage of fluid from the fluid source 64 to the third tract 69 is prevented. On the contrary, in the second operating position, the passage of fluid from the fluid source 64 to the third tract 69 is allowed, while the passage of fluid from the fluid source 64 to the second tract 68 is prevented. Notably, the first operating position and the second operating position may be taken during a standard dialysis treatment to either pre- or post-infuse a replacement fluid into the extracorporeal blood circuit, while the recirculation position of selector 70 is taken to allow fluid recirculation in the recirculation condition.

Selector 70 may consist of a valve element operating on the infusion line 63 by alternatively blocking the passage of fluid in either branch and further placeable in the neutral position. Suitable selectors may be alternatively provided, which are able to establish a priori the amount of liquid that has to pass through both branches simultaneously. It will also be possible to vary the percentages of fluid in either branch as a function of time and of the pre-established therapies. Notably the selector 70 is connected to the control unit 12, wherein the latter is configured to control selectively the selector in said first operating position, in the second operating position and in the recirculation position.

The disposable set may further comprise a pre-blood-pump (PBP) infusion line 51 extending from a first end 51a for connection to a second fluid source 10, and a second end 51b fluidly connected to the blood withdrawal line 6. The pre-blood-pump infusion line 51 may be an anticoagulant infusion line 51; in such a case the second fluid source 10 may house a fluid solution including citrate and/or citric acid. The second fluid source 10 may be a plastic bag housing the PBP infusion fluid. The second end 51b of the pre-blood-pump infusion line 51 is interposed between the second end 6b of the blood withdrawal line 6 and the blood pump tract 6p as shown in figures 1 and 2. Further, the second end 51b of the pre-blood-pump infusion line 51 may be interposed between the second end 6b of the blood withdrawal line 6 and the first site 31 as shown in figure 1 in case of direct connection of the infusion line 63 to the blood withdrawal line 6. Indeed, in such a case, connection between second end 63b of the infusion line 63 and the withdrawal line is optionally downstream the second end 51b of the pre-blood-pump infusion line 51. The pre-blood-pump infusion line 51 also comprises a respective PBP (pre-blood-pump) infusion pump tract 51p configured to be engaged by a respective PBP infusion pump 54 configured to infuse the substance from the second fluid source 10 through the pre-blood-pump infusion line 51 and into the blood circuit 17.

The PBP infusion pump 54 is operatively connected to the control unit 12 which is configured to selectively control the PBP infusion pump 54 to promote the infusion fluid to flow within the infusion line 51 and to deliver the infusion fluid into the blood withdrawal line 6: the control unit 12 may be configured to control the PBP infusion pump 54 to set the prescription flow rate. Also in this regard, the type of treatment, the infusion bag content, and other conditions determine the set infusion rate. The control unit may also be configured to stop the PBP infusion pump 54 to arrest delivery of the infusion fluid.

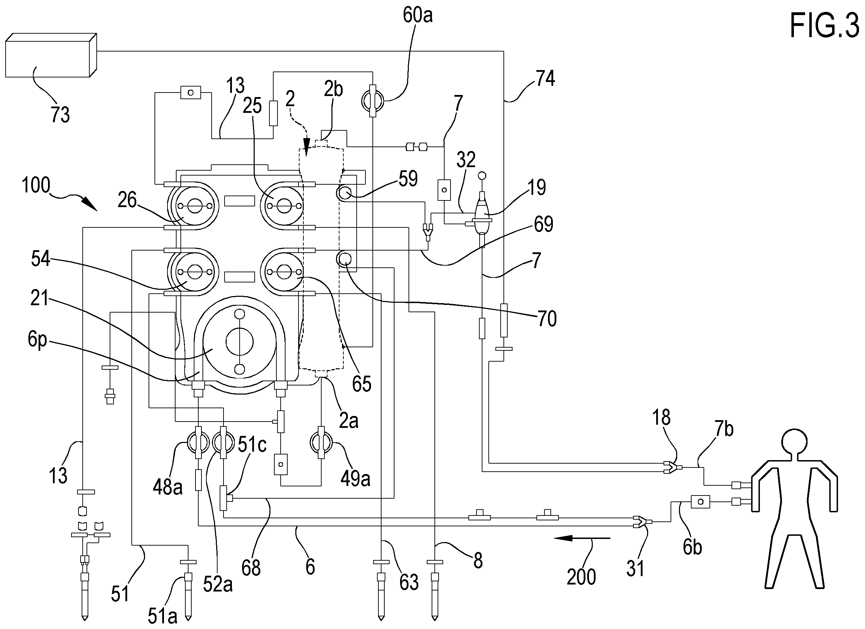

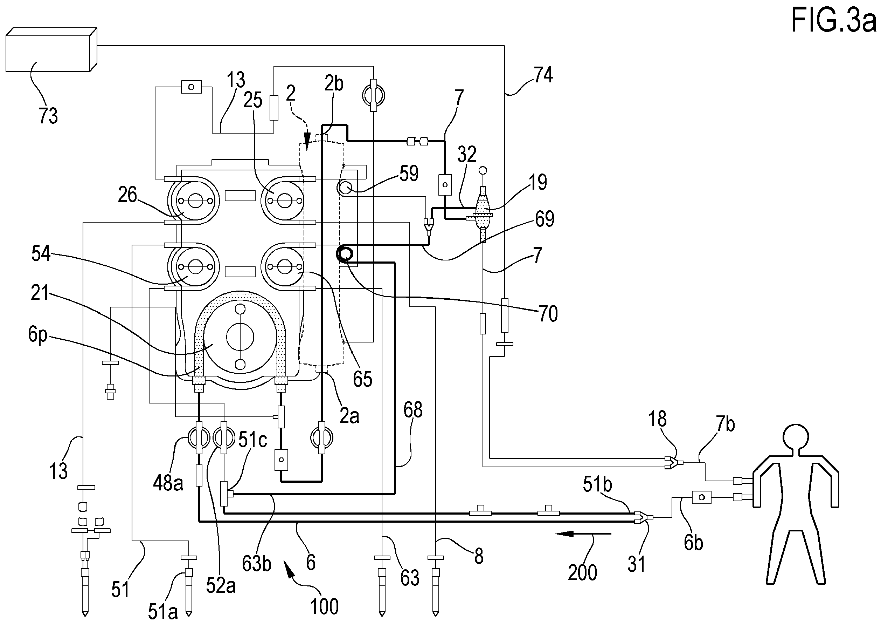

According to the embodiment of figures 2 and 3, the second end 51b of the pre-blood-pump infusion line 51 is connected to the blood withdrawal line 6 at the first site 31. In this case, the second tract 68 of the infusion line 63 is directly connected to the pre-blood-pump infusion line 51 at an access point 51c interposed between the second end 51b of the pre-blood-pump infusion line 51 and the PBP infusion pump tract 51p of the pre-blood-pump infusion line 51. In other terms, the infusion line 63 and the pre-blood-pump infusion line 51 are connected together, at the access point 51c, and fluid moves towards the blood withdrawal line 6 along a common infusion tract extending up to the first site 31 (i.e., the common infusion tract runs from the access point 51c to the second end 51b of the pre-blood-pump infusion line 51).

The disposable set further comprises an ion replacement fluid line 74 connected, on one end, to an ion replacement container 73 and, to the other end, to the blood return line (7), for example at an access site 18, such as a connector allowing infusion and homogenous mixing of the infused fluid with the blood. As shown in the enclosed figures, the access site 18 is placed downstream the second site 32 and possibly, but not necessarily, upstream the air bubble sensor 55. An ion replacement pump 75 acting on a ion replacement pump tract 75p allow to pump the fluid through the ion replacement infusion line 74. In an alternative embodiment (not shown) the ion replacement infusion line 74 is directly connected to an additional patient vascular access directly infusing the ion replacement fluid into the patient blood (e.g., into a venous peripheral vessel of the patient).

Usually, the ion replacement infusion line 74 is used to infuse a calcium (and possibly a magnesium, too) concentrated solution to re-establish the proper electrolyte balance in the patient blood during treatments with regional anticoagulation using citrate.

According to the disclosed embodiments, the disposable set 100 further comprises a fluid supply line 8. The fluid supply line 8 delivers dialysis fluid from a dialysis liquid source 14 to the filtration unit 2. The dialysis liquid source 14 consists of one or more bags containing a suitable dialysis liquid designed to supply the supply line 8 of the dialysis fluid circuit 16 with dialysis fluid. The dialysis liquid supply line 8 also comprises a respective supply pump tract 8p configured to be engaged by a dialysis fluid pump 25 of the extracorporeal blood treatment apparatus to promote dialysis fluid flow towards the fluid inlet of the secondary chamber 4 of the filtration unit.

The dialysis fluid pump 25, i.e. a peristaltic pump, defines a direction 200 of dialysis fluid circulation and controls the flow rate of the fresh dialysis liquid from the bag 64 towards the filtration unit 2.

In the embodiments of the figures, downstream from the dialysis fluid pump 25 in the direction of circulation 200 a branching 56 is provided splitting the dialysis supply line 8 up into an intake branch 57 and a post infusion tract 58. In other terms, the supply line 8 splits in a post infusion tract 58 extending up to the return line 7 so that the fluid supply line 8 is fluidly connected to the return line 7 of the blood circuit 17.

By means of said infusion branch 58 it is possible to obtain a post-infusion directly in the blood line 17 using the content of the primary fluid container 64. The post infusion tract 58 may share an end portion with the third tract 69 of the infusion line 63, so that both infusion line 63 and the post infusion tract 58 are connected together and deliver fluid to the second site 32. In particular, the post infusion line 63 and the post infusion tract 58 may be connected to the bubble trap 19 of the disposable set. The shared end portion is interposed between the point wherein the infusion line 63 joins the post infusion tract 58 and the second site 32/the bubble trap 19.

In a different embodiment (not shown), the post infusion tract 58 may extend directly to the bubble trap 19 or to the blood return line 7, without sharing any portion with the infusion line 63.

Conversely, the intake branch 57 conveys the fluid directly to the filtration unit 2 and in particular to the secondary chamber 4 of said filtration unit. The dialysis fluid circuit 16 is further equipped with a selector 59 for determining the percentages of fluid flow within the infusion branch 58 and the intake branch 57. Generally said selector 59, usually placed near the branching 56, can be positioned at least between a first operating position in which it allows the passage of fluid in the intake branch 57 and blocks the passage in the post infusion line 58, and a second operating position in which it allows the passage of fluid in the post infusion line 58 and blocks the passage in the intake branch 57. In other words, said selector 59 may consist of a valve element operating on the dialysis fluid circuit 16 by alternatively blocking the passage of fluid in either branch. Suitable selectors may be alternatively provided, which are able to establish a priori the amount of liquid that has to pass through both branches simultaneously. It will also be possible to vary the percentages of fluid in either branch as a function of time and of the pre-established therapies. Notably the selector 59 may be connected to the control unit 12, wherein the latter is configured to control selectively the selector in said first operating condition and in the second operating condition.

The dialysis liquid through the intake branch 57 gets into the secondary chamber 4 of the filtration unit 2. In particular, the primary chamber 3 through which the blood flow passes is separated from the secondary chamber 4 through which the dialysis liquid passes by means of the semipermeable membrane 5 ensuring the suitable passage of the substances/molecules and of fluid from the blood towards the dialysis liquid by means of convection and diffusion processes, and further ensuring through diffusion the passage of substances/molecules from the dialysis liquid towards the blood.

The disposable set 100 further comprises an effluent fluid line 13 connected to the fluid outlet of the filtration unit 2: the effluent fluid line 13 may also comprise a respective effluent pump tract 13p configured to be engaged by an effluent pump 26. The fluid to be removed then passes through an effluent pressure sensor 60 and a blood detector 61 and is conveyed into a drain, such as a collection container or bag 62. An actuator is provided for conveying fluid, for instance an effluent pump 26 controlling the flow rate in the effluent fluid line 13 within the fluid circuit 16. Also said pump is generally a peristaltic pump.

The disposable set 100 may further comprise one or more pressure detecting sites 48a, 49a, 50a, 52a, 60a (see figures 3 to 5) arranged on the blood circuit 17 and/or on the infusion line 63 and/or on the pre-blood-pump infusion line 51 and/or on the effluent line 13; the pressure detecting sites allow detecting a pressure in the respective line/circuit portion. In general, the pressure detecting sites 48a, 49a, 50a, 52a, 60a may be in the form of PODs (pressure oscillating diaphragms) that, once connected to the respective sensors on the extracorporeal blood treatment apparatus, allow to measure the pressure in the tube portion where the POD is placed. PODs placed on the blood circuit (or on tube section where blood may flow) are designed to eliminate the blood air interface, thereby reducing the risk of blood clotting.

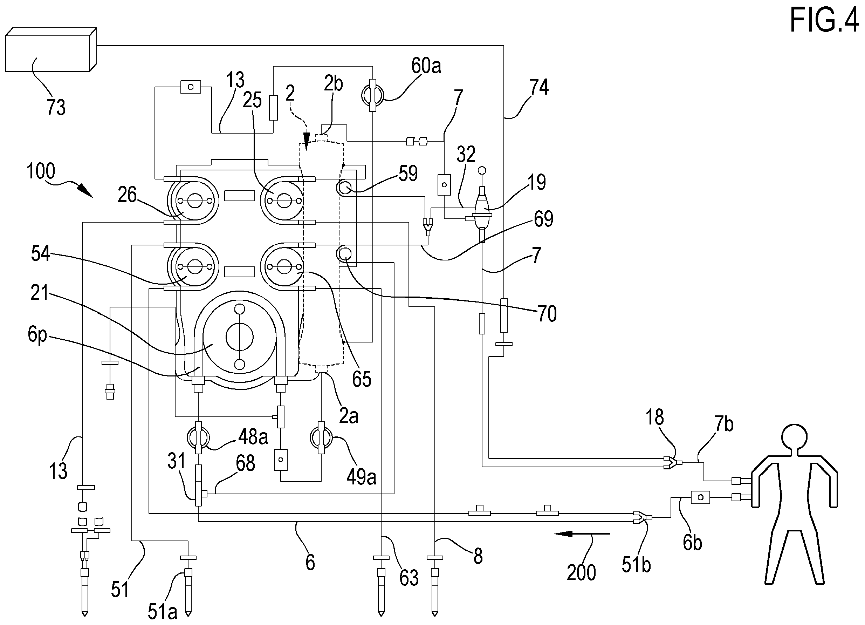

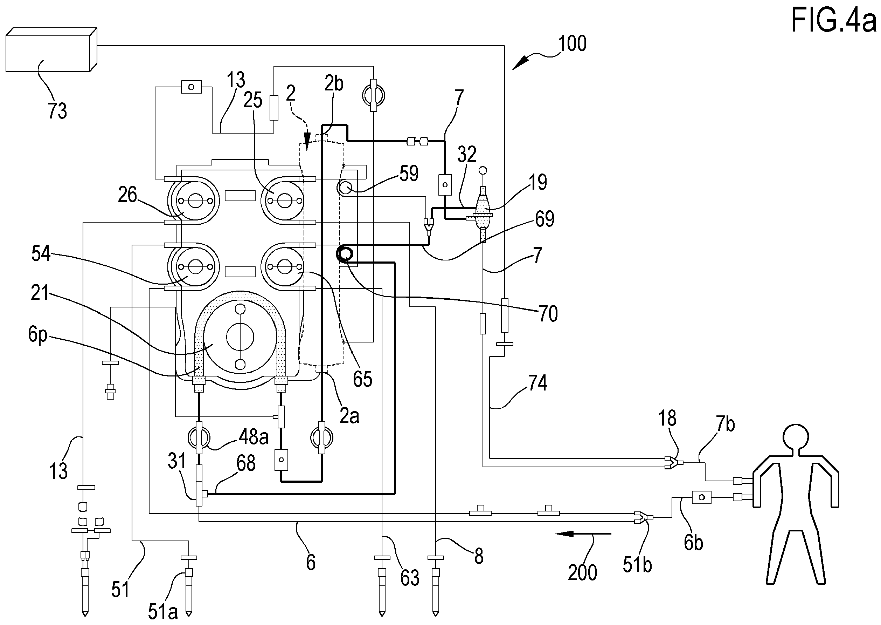

For example, a pressure detecting site 48a (e.g., a POD) may be arranged on the blood withdrawal line 6 between the first site 31 and the blood pump tract 6p, as shown in figures 3, 3a, 4, and 4a. An (additional) pressure detecting site 49a (e.g., a POD) may be arranged on the blood withdrawal line 6 between the blood pump tract 6p and the blood inlet 2a of the filtration unit 2. This is shown in all the figures 3 to 5.

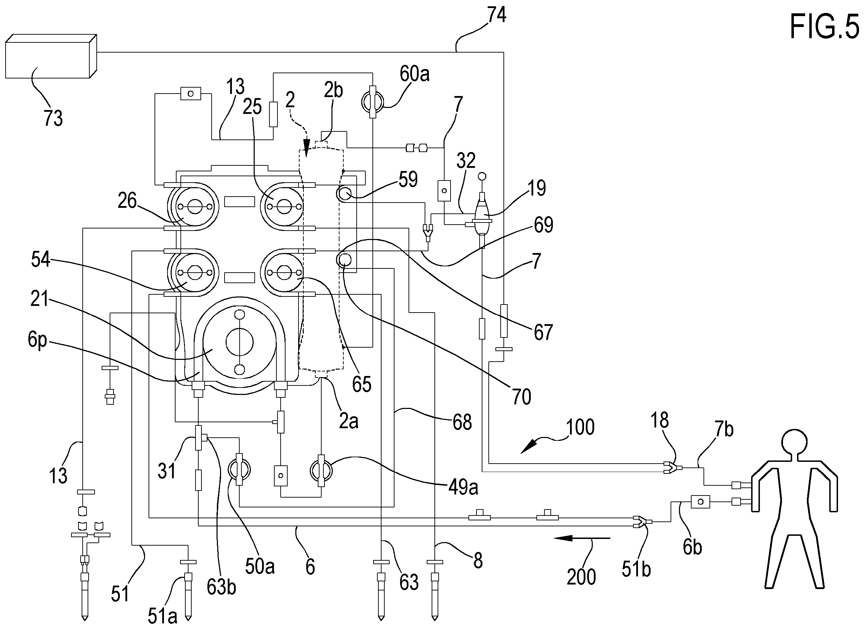

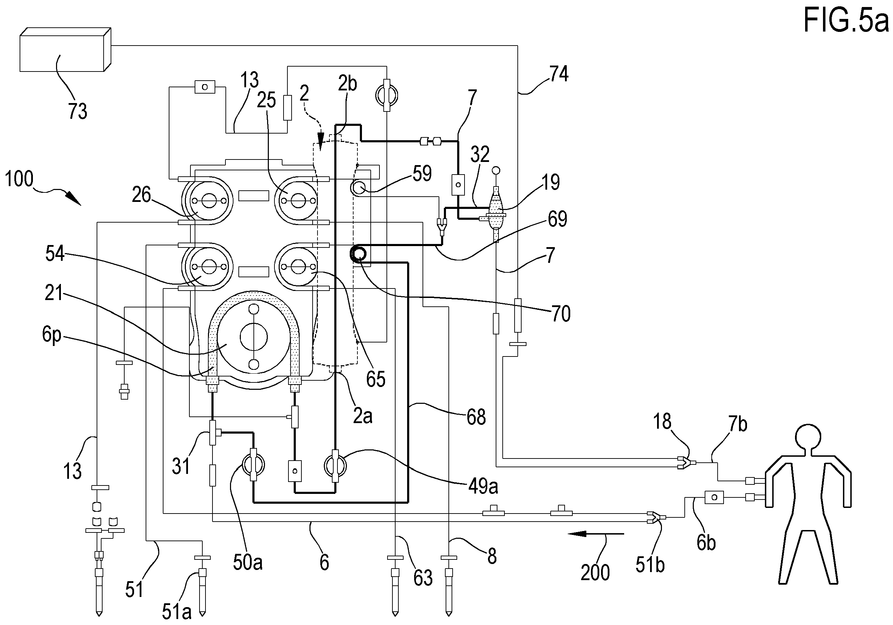

Alternatively to pressure detecting site 48a (or, in a non-shown embodiment, in addition to), a pressure detecting site 50a (e.g., a POD) may be arranged on the infusion line 63 close to, or at, the first site 31 (see figures 5 and 5a). Alternatively to pressure detecting site 48a on the blood withdrawal line 6 or pressure detecting site 50a on the infusion line 63 (or, in a non-shown embodiment, in addition to one or both), a pressure detecting site 52a (e.g., a POD) may be arranged on the PBP infusion line 51 (see figures 3 and 3a). The pressure detecting site of the disposable set may further comprise itself a pressure gauge, or alternatively may be configured to be connected to a pressure gauge configured to measure the blood or fluid pressure in the respective line.

Indeed, when the respective pressure site of the disposable set is properly connected to the pressure gauge of extracorporeal blood treatment apparatus, it defines a respective pressure sensor. In this regard, according to figures 1 and 1a, a first pressure sensor 48 is defined just upstream the blood pump 21 and a second pressure sensor 49 is defined downstream the blood pump 21 and upstream the filtration unit 2. An effluent sensor 60 is located in the effluent line 13 downstream the filtration unit 2 and upstream the effluent pump 26.

In the embodiment of figures 2 and 2a, pressure sensor 48 is removed and an additional pressure sensor 52 is located on the PBP infusion line 51 upstream the junction point 51c and downstream the PBP pump 54.

As an alternative to placing the sensor on the PBP infusion line 51, a pressure sensor 50 could be directly placed on the infusion line 63 and in particular at and immediately upstream of the junction point 51c. This latter configuration is shown in dashed lines. The control unit 12 is connected to all sensor, actuators, and pumps and controls the operations of the apparatus.

### PRE-POST RECIRCULATION SEQUENCE

The blood circuit 17 may be configured in a loop recirculation circuit 11 wherein blood may be recirculated in case e.g., an alarm is issued or e.g., an event occurs. From the structural point of view, in a first embodiment (e.g., figure 1a), the loop recirculation circuit 11 includes a delivery portion of the blood withdrawal line 6 interposed between the first site 31 of the blood withdrawal line 6 and the blood inlet 2a of the filtration unit 2 (and including the blood pump tract 6p), the blood chamber of the filtration unit 2, a receiving portion of the blood return line 7 interposed between the blood outlet 2b of the filtration unit 2 and the second site 32 of the blood return line 7 and a recirculation portion 66 of the infusion line 63 interposed between the first site 31 and the second site 32. More in detail, the recirculation portion 66 of the infusion line 63 comprises the second tract 68 and the third tract 69 of the infusion line 63 that are connected to each other at the branch site 67. The loop recirculation circuit 11 may further comprise the bubble trap 19 in case the third tract 69 enters into the bubble trap 19 for infusing into the blood return line 7. See figures 1a, 4a, and 5a.

In figure 2a, the loop recirculation circuit 11 includes a delivery portion of the blood withdrawal line 6 interposed between the first site 31 of the blood withdrawal line 6 and the blood inlet 2a of the filtration unit 2 (and including the blood pump tract 6p), the blood chamber of the filtration unit 2, a receiving portion of the blood return line 7 interposed between the blood outlet 2b of the filtration unit 2 and the second site 32 of the blood return line 7 and a recirculation portion 66 of the infusion line 63 interposed between the first site 31 and the PBP infusion line 51. More in detail, the recirculation portion 66 of the infusion line 63 comprises the second tract 68 and the third tract 69 of the infusion line 63 that are connected to each other at the branch site 67. Moreover, the loop recirculation circuit 11 comprises the common tract of the PBP infusion line 51 between the access point 51c and the second end 51b of the PBP infusion line 51. The loop recirculation circuit 11 may further comprise the bubble trap 19 in case the third tract 69 enters into the bubble trap 19 for infusing into the blood return line 7. See figures 2a and 3a. During a recirculation condition, the loop recirculation circuit 11 is configured to allow blood to continuously flow within the loop recirculation circuit 11 in loop: the blood pump 21, engaged to the blood pump tract 6p, is configured to determine the blood flow within the loop recirculation circuit 11. The loop recirculation circuit is shown, with bold lines, in figures 1a, 2a, and according to specific embodiments of the disposable set 100, in figures 3a, 4a, and 5a. The loop recirculation circuit 11 is configured to allow fluid recirculation, in a clockwise and/or in a counter-clockwise direction, in order to avoid blood clogging in a situation wherein for example the blood pump has been temporarily stopped and the patient isolated as a reaction to an alarm or an event.

In general, blood recirculation in the loop recirculation circuit 11 may be started without stopping the blood pump 21. Indeed, it is sufficient that the control unit 12 sets the disposable in a configuration that allows the blood pushed by the blood pump 21 to recirculate in the loop recirculation circuit 11. It might be sufficient to allow blood to flow along the third tract 69 and the second tract 68 of the infusion line 63. To achieve this configuration, the selector 70 is moved to the recirculation position (neutral position). No infusion of fluid through infusion line 63 is commanded (i.e., infusion pump 65 is stopped). Notably, the blood pump speed may be different during the recirculation procedure with respect to the blood pump speed during extracorporeal blood treatment.

As apparent, to achieve the blood recirculation in the loop recirculation circuit 11 it is not necessary to close any of the safety valves 20 and 27. Indeed, the safety valve/s 20, 27 on the blood circuit 17 may also be left open in case of total or partial access obstruction scenario; the control unit 12 still controls the blood pump 21 to determine blood flow within the loop recirculation circuit 11 of the blood circuit 17 of the disposable set, but additionally (in case of partial occlusion) a blood circulation is maintained in the blood circuit section between second site 32 and the patient and between the patient and the first site 31. Indeed, in the latter scenario, where the obstruction is not 'full/total', a small blood flow 'q' could be taken from the catheter at the second end 6b and the same small blood flow is returned to second end 7b, while a recirculation flow 'Q_{b}-q' (difference from the blood flow Qb defined by the blood pump action and the residual small flow q to and from the patient access) is present in the infusion line branches, namely in the second and third tracts 68 and 69.

Clearly, the alarm condition may trigger the need of blood pump stop and/or safety valve/s closure. Differently, blood pump stop may not be a must in a scenario where the recirculation is selected by the operator (versus alarm driven). In this case, it may be considered opening the intercepting element 70 before - optionally - close safety valves 20 and/or 27, and without stopping the blood pump 21.

Thus, the provided configuration allows keeping blood in movement, while the patient, if connected to the disposable set, may, or may not be isolated.

In order to allow recirculation of the fluid/blood in the loop recirculation circuit, the disposable set should not comprise or implement any component that may prevent recirculation, such as one-way valves in the infusion line/s, clamps, or selectors preventing or configured to prevent the blood flow in the loop.

According to the embodiments of figures 1, 1a, 4, 4a, and 5, and 5a the infusion line 63 is directly connected to the blood withdrawal line 6 at the first site 31. The term "*directly*" specifies that the infusion line 63 has its second end 63b fixed to the blood withdrawal line 6. Further, further branches are absent on the infusion line 63 between the infusion site 31 and the branch site 67.

According to the embodiments of figures 2, 2a, 3 and 3a, the infusion line 63 is not directly connected to the withdrawal line 6: the infusion line 63 shares indeed a common end portion with the pre-blood-pump infusion line 51. Thus said common end portion is configured to selectively transport the fluid solution coming from either the second fluid source 10, the fluid solution coming from the infusion fluid source 64, or blood during the blood recirculation condition. During the recirculation condition, safety clamp/s 20, 27 on the blood withdrawal line 6 and/or on the blood return line 7 are optionally closed.

A default sequence may be initiated from an alarm state where blood pump is stopped and safety valve 20 or clamp on the blood return line 7 closed. In general, the infusion fluid source 64 (solution bag) should be available for the infusion line 63 e.g., on the replacement scale C. The sequence is very similar for all the shown embodiments.

The main steps of the sequence are the following: the control unit 12 moves the selector 70 to the recirculation position (neutral position); before moving the selector 70, the control unit 12 checks the bubble trap 19 fluid level and checks the access and return pressures to verify that they are compatible with moving the selector 70 to neutral position. If necessary, blood or fluid level in the bubble trap 19 is adjusted using the air pump 47.

Before re-starting the blood pump at a default low flow rate (usually different from the blood pump set during the extracorporeal blood treatment - blood flow rate is set from configuration menus), the control unit 12 checks the blood level in the bubble trap 19 to raise it in order to avoid recirculating air bubbles.

Note that in case vascular access to the patient (e.g., catheter access way) is obstructed, there might be a large pressure difference across the blood pump when the flow is stopped. Similar situation may occur during high flow rate therapies (such as ECCO2R) in the case where safety valves 20 and/or 27 are closed.

In case of large pressure differences across the blood pump, when selector 70 is suddenly moved to neutral, air bubbles may be drawn into the third tract 69 of the infusion line 63 (even if fluid level in the bubble trap 19 was OK before the selector 70 switch). To avoid the problem, the pressure difference (access vs return pressure) should be - at least partly - equalized before the selector 70 is moved.

Clearly, opening of safety valves 20 and 27 is an easy way, if considered safe given the occurring situation. However, in case of full vascular access obstruction, it might not work. As an alternative, the control unit 12 may infuse some infusion fluid e.g., from infusion fluid source 64 via infusion line 63 and/or from dialysis liquid source 14 via supply line 8 and post infusion line 58 or also across the filtration unit 2 towards the blood circuit 17 to adjust at least one of the access and return pressures as to bring them to a closer value range (e.g. within less than 50 or 100 mmHg of pressure difference). In this situation, the pressure equalization is a prerequisite to the move of the selector 70, rather than to the restart of the blood pump.

During the pre-post recirculation, the control unit 12 runs the blood pump usually, but not necessarily, in the same direction as during treatment. In particular, the control unit 12 keeps the blood pump running, while the operator intervenes and performs the troubleshooting.

Once operator intends to resume treatment, the control unit 12 asks for confirmation to resume the treatment or to change/discard the disposable set.

Once the operator confirms to resume treatment, the control unit 12 checks the blood level in the bubble trap 19 and provides an adjustment (e.g., via air pump 47) according to pressure level during recirculation versus last return pressure operating point prior to the recirculation.

Subsequently, the control unit 12 switches the selector 70 to pre-infusion position (first operating position) before restarting all pumps to their respective prescription settings. The control unit 12 controls the blood pump 21 to reach the prescribed blood flow rate.

Further, the control unit 12 is configured to perform a rinsing step of infusion line 63, and in particular of the second tract 68 of the infusion line 63 where blood was recirculated. In case the pre-infusion flow rate is too low for timely rinsing (or absent in the treatment settings, i.e., no pre-infusion), the control unit 12 commands a specific (higher) flow rate through the second tract 68 of the infusion line 63. Further, also the third tract 69 of the infusion line 63 is rinsed. The control unit 12 is configured to move the selector 70 to post-infusion (second operating position) and to wash blood away from the third tract 69.

Finally, in case the circuit according to figure 2 and 2a is used for recirculation, then also PBP line should be rinsed. In case PBP flow rate is not sufficient for rinsing, then the control unit 12 is configured to set a flow rate in the PBP line sufficient for the complete rinsing. Eventually, PBP flow rate and pre-infusion flow rate for rinsing are synchronized so that the common line tract is fully rinsed (e.g., without infusing too much citrate solution).

Of course, if therapy prescription provides for post-infusion rather than pre-infusion, the control unit 12 switches the selector 70 to the second condition (post-infusion) after completion of the previously described rinsing steps.

## Claims

1. Disposable set (100) for an extracorporeal blood treatment apparatus (1) comprising:
- a filtration unit (2) having a blood inlet (2a) and a blood outlet (2b);
- a blood circuit (17) including:
➢ a blood withdrawal line (6) extending between a first end (6a) connected to the blood inlet (2a) of the filtration unit (2), and a second end (6b) for direct or indirect connection to a patient (P);
➢ a blood return line (7) extending between a first end (7a) connected to the blood outlet (2b) of the filtration unit (2), and a second end (7b) for direct or indirect connection to the patient;
➢ a blood pump tract (6p) located on the blood circuit (17) and configured to be engaged to a blood pump (21) of the extracorporeal blood treatment apparatus to promote blood flow in the blood circuit,
- an infusion line (63) extending from a first end (63a) for connection to an infusion fluid source (64) and branching off at a branch site (67) towards:
➢ a second end (63b) of the infusion line (63) in direct fluid communication with the blood withdrawal line (6) at a first site (31), the first site (31) being interposed between the second end (6b) of the blood withdrawal line (6) and the blood pump tract (6p) and the second end (63b) of the infusion line (63) is directly connected to the blood withdrawal line (6) at the first site (31); and
➢ a third end (63c) of the infusion line (63) in direct or indirect fluid communication with the blood return line (7) at a second site (32), the second site (32) being interposed between the blood pump tract (6p) and the second end of the blood return line (7);
wherein the blood circuit is designed to allow fluid flow in a loop recirculation circuit (11) including:
- a portion of the blood withdrawal line (6) interposed between the first site (31) of the blood withdrawal line (6) and the blood inlet (2a) of the filtration unit (2);
- the blood pump tract (6p);
- the filtration unit (2);
- a portion of the blood return line (7) interposed between the blood outlet (2b) of the filtration unit (2) and the second site (32) of the blood return line (7);
- a recirculation portion (66) of the infusion line (63) downstream of the branch site (67) and interposed between the first site (31) and the second site (32);
the loop recirculation circuit (11) being configured to allow blood or a fluid, at least in a recirculation condition, to continuously flow in a loop circulation.

2. Disposable set according to claim 1, wherein the loop recirculation circuit (11) is configured to allow fluid recirculation in a first direction from the first site (31) towards the filtration unit (2) and from the filtration unit (2) towards the second site (32) or in a second direction from the second site (32) towards the filtration unit (2) and from the filtration unit (2) towards the first site (31), and wherein:
- the blood inlet (2a) of the filtration unit (2) is in bidirectional fluid communication with the recirculation portion (66) of the infusion line (63) through the first site (31); and
- the blood outlet (2b) of the filtration unit (2) is in bidirectional fluid communication with the recirculation portion (66) of the infusion line (63) through the second site (32).

3. Disposable set according to any one of claims 1 and 2, wherein
the first site (31) comprises a fluid connector, in particular a three-way fluid connector, including:
- a blood inlet fluidly connected to the withdrawal line (6) and facing the second end (6b) of the blood withdrawal line (6);
- a blood outlet fluidly connected to the withdrawal line (6) and facing the first end (6a) of the blood withdrawal line (6), optionally facing the pump tract (6p) of the blood withdrawal line (6);
- an infusion inlet fluidly connected to the recirculation portion (66) of the infusion line (63), and in particular fluidly connected to the second end (63b) of the infusion line (63).

4. Disposable set according to any one of the preceding claims 1 to 3, further comprising a blood warmer disposable unit comprising a blood flow path with an inlet and an outlet, the blood warmer disposable unit being fluidly connected to the blood circuit (17) upstream of the second site (32),
wherein the blood warmer disposable unit is included in the loop recirculation circuit (11),
in particular, the inlet of the blood warmer disposable unit being fluidly connected to the blood return line (7) and the outlet of the blood warmer disposable unit being fluidly connected to the blood return line (7) upstream of the second site (32), and upstream of a bubble trap (19).

5. Disposable set according to any one of claims 1 to 4, wherein the infusion line (63) extends from the first end (63a) of the infusion line (63) for a first tract (72), said first tract (72) branching at the branch site (67) into:
- a second tract (68) extending from the branch site (67) up to the second end (63b) of the infusion line (63); and
- a third tract (69) extending from the branch site (67) up to the third end (63c) of the infusion line (63),
the recirculation portion (66) of the infusion line (63) comprising the second tract (68) and the third tract (69) of the infusion line (63),
wherein the infusion line (63) comprises a respective infusion pump tract (65p) arranged on the first tract (72) of the infusion line (63) and configured to be engaged by an infusion pump (65) of the extracorporeal blood treatment apparatus, said infusion pump tract (65p) being interposed between the first end (63a) and the branch site (67) of the infusion line (63).

6. Disposable set according to any one of claims 1 to 5, wherein the first site (31) is arranged on the blood withdrawal line (6) upstream of the blood pump tract (6p) with respect to a blood withdrawal direction (200) directed from the second end (6b) to the first end (6a) of the blood withdrawal line (6), in particular the first site (31) is closer to the second end (6b) of the blood withdrawal line (6) than to the first end (6a) of the blood withdrawal line (6), and wherein the third end (63c) of the infusion line (63) is directly connected to the blood return line (7) at the second site (32).

7. Disposable set according to any one of claims 1 to 6, further comprising:
- a safety valve (27) placed on the blood withdrawal line (6) between the second end (6b) of the blood withdrawal line (6) and the first site (31),
- a safety valve (20) placed on the blood return line (7) between the second site (32) and the second end (7b) of the blood return line (7),
wherein the loop recirculation circuit (11) is a closed circuit.

8. Disposable set according to any one of claims 1 to 7, wherein the filtration unit (2) comprises:
➢ a primary chamber (3) fluidly connected to the blood circuit through the blood inlet and the blood outlet of the filtration unit (2),
➢ a secondary chamber (4) fluidly connected or connectable to a fluid circuit (16), said secondary chamber (4) being configured to receive dialysis fluid and/or to remove effluent fluid,
and wherein the primary chamber (3) is separated from the secondary chamber (4) by a semi-permeable membrane (5),
the disposable set further comprising a fluid circuit (16) including:
- an effluent fluid line (13) extending between a first end for connection to a discharge unit, such as a collection container (62), and a second end fluidly connected to a fluid outlet of a secondary chamber (4) of the filtration unit (2), wherein said effluent fluid line (13) comprises an effluent pump tract (13p) configured to be engaged to an effluent pump (26) of the extracorporeal blood treatment apparatus to promote flow of effluent fluid from the fluid outlet of the secondary chamber (4) towards the discharge unit;
- optionally a fluid supply line (8) extending between a first end for connection to a dialysis fluid source (14), and a second end fluidly connected to a fluid inlet of a secondary chamber (4) of the filtration unit (2), wherein said fluid supply line (8) comprises a supply pump tract (8p) configured to be engaged to a supply pump (25) of the extracorporeal blood treatment apparatus to promote dialysis fluid towards the fluid inlet of the secondary chamber (4) of the filtration unit (2).

9. Extracorporeal blood treatment apparatus including:
- one or more pumps (21, 25, 26, 54, 65, 75) comprising a blood pump (21),
- one or more intercepting elements (20, 27, 59, 70),
- a disposable set (100), particularly in accordance with any one of the previous claims, comprising:
➢ a filtration unit (2) having a blood inlet (2a) and a blood outlet (2b);
➢ a blood circuit (17) including:
▪ a blood withdrawal line (6) extending between a first end (6a) connected to the blood inlet (2a) of the filtration unit (2), and a second end (6b) for connection to a patient (P);
▪ a blood return line (7) extending between a first end (7a) connected to the blood outlet (2b) of the filtration unit (2), and a second end (7b) for connection to the patient;
▪ a blood pump tract (6p) located on the blood circuit (17) and engaged to the blood pump (21) of the extracorporeal blood treatment apparatus to promote blood flow in the blood circuit,
➢ an infusion line (63) extending from a first end (63a) for connection to an infusion fluid source (64) and branching off at a branch site (67) towards:
▪ a second end (63b) of the infusion line (63) in direct fluid communication with the blood withdrawal line (6) at a first site (31), the first site (31) being interposed between the second end (6b) of the blood withdrawal line (6) and the blood pump tract (6p) and the second end (63b) of the infusion line (63) is directly connected to the blood withdrawal line (6) at the first site (31); and
▪ a third end (63c) of the infusion line (63) in direct or indirect fluid communication with the blood return line (7) at a second site (32), the second site (32) being interposed between the blood pump tract (6p) and the second end of the blood return line (7);
- a control unit (12) configured to control said one or more pumps (21, 25, 26, 54, 65, 75) and said one or more intercepting elements (20, 27, 59, 70) in order to define different flow circuits inside the blood circuit (17) and the infusion line (63), the control unit (12) being configured to define at least the following flow circuits:
➢ a treatment flow circuit, wherein the one or more pumps (21, 25, 26, 54, 65, 75) and the one or more intercepting elements (20, 27, 59, 70) are configured to allow blood to flow from the second end (6b) of the blood withdrawal line (6) to the filtration unit (2) and from the filtration unit (2) to the second end (7b) of the blood return line (7); and
➢ a loop recirculation circuit (11), wherein the one or more pumps (21, 25, 26, 54, 65, 75) and the one or more intercepting elements (20, 27, 59, 70) are configured to allow blood or a fluid to continuously flow in a loop circulation,
wherein the loop recirculation circuit (11) includes:
• a portion of the blood withdrawal line (6) interposed between the first site (31) of the blood withdrawal line (6) and the blood inlet (2a) of the filtration unit (2);
• the blood pump tract (6p);
• the filtration unit (2);
• a portion of the blood return line (7) interposed between the blood outlet (2b) of the filtration unit (2) and the second site (32) of the blood return line (7);
• a recirculation portion (66) of the infusion line (63) downstream of the branch site (67) and interposed between the first site (31) and the second site (32).

10. Apparatus according to claim 9, wherein the control unit (12) is configured to perform a recirculation procedure comprising the steps of:
- defining the loop recirculation circuit (11) by at least:
➢ commanding the one or more intercepting elements (20, 27, 59, 70) to put the first site (31) in fluid communication with the second site (32) through the recirculation portion (66) of the infusion line (63);
- controlling the blood pump (21) to determine blood flow within the loop recirculation circuit (11) of the blood circuit (17) of the disposable set.

11. Apparatus according to any one of claims 9 and 10, wherein, the control unit (12) is configured to stop the blood pump (21) before starting the recirculation procedure, and, during the recirculation procedure, the control unit (12) is configured to (i) stop any ultrafiltration of fluids from a primary chamber (3) towards a secondary chamber (4) of the filtration unit (2), (ii) keep the patient (P) fluidly isolated from the loop recirculation circuit (11), and (iii) recirculate blood in the loop recirculation circuit (11) to prevent blood from clotting.

12. Apparatus according to any one of the preceding claims 9 to 11, wherein one or more pumps (21, 25, 26, 54, 65, 75) comprise an infusion pump (65) active on the infusion pump tract (65p) of the infusion line, wherein, in the recirculation procedure, the control unit (12) is configured to:
a. keep said infusion pump (65) inactive, in particular the infusion pump (65) being an occlusive pump, such as a peristaltic pump;
b. run the blood pump (21) at a speed different from a speed of the blood pump (21) during the extracorporeal blood treatment, in particular the control unit (12) is configured to run the blood pump (21) in a same direction during the recirculation procedure as during the extracorporeal blood treatment.

13. Apparatus according to any one of the preceding claims 9 to 12, wherein the disposable set comprises a blood warmer disposable unit having a blood flow path with an inlet and an outlet, the blood warmer disposable unit being fluidly connected to the blood circuit (17) upstream of the second site (32), the inlet of the blood warmer disposable unit being fluidly connected to the blood return line (7) and the outlet of the blood warmer disposable unit being fluidly connected to the blood return line (7) upstream of the second site (32),
wherein during the recirculation procedure, the control unit (12) is configured to:
• recirculate the blood contained in the blood warmer bag; and either
∘ stop heating the blood warmer bag; or
∘ adjust a set point temperature of a warmer unit as a function of a patient actual blood temperature or of a patient desired blood temperature or as a default setting.

14. Apparatus according to any one of the preceding claims 9 to 13, wherein the control unit (12) is configured to:
a. end the recirculation procedure in case an elapsed recirculation time is higher than a pre-set recirculation time, optionally wherein the pre-set recirculation time is a function of the anticoagulation mode during extracorporeal blood treatment;
b. request confirmation to a user, e.g., via a user interface, to resume the treatment once the recirculation procedure is ended.

15. Apparatus according to any one of the preceding claims 9 to 14, wherein the control unit (12) is configured to perform a recirculation procedure comprising the steps of defining the loop recirculation circuit (11) by at least:
• commanding the one or more intercepting elements (20, 27, 59, 70) to prevent fluid passage either or both in the blood withdrawal line (6) between the second end (6b) of the blood withdrawal line (6) and the first site (31) and in the blood return line (7) between the second site (32) and the second end (7b) of the blood return line (7).
